(19) Europäisches Patentamt
European Patent Office
Office européen des brevets

(11) Numéro de publication: **0 317 427 B1**

(12) # FASCICULE DE BREVET EUROPEEN

(45) Date de publication de fascicule du brevet: **12.01.94**

(51) Int. Cl.[5]: **C07D 461/00**, A61K 31/435

(21) Numéro de dépôt: **88402872.1**

(22) Date de dépôt: **16.11.88**

Le dossier contient des informations techniques présentées postérieurement au dépôt de la demande et ne figurant pas dans le présent fascicule.

(54) **Nouveaux dérivés substitués de 20,21-dinoréburnaménine,leur procédé de préparation et les nouveaux intermédiaires ainsi obtenus, leur application comme médicaments et les compositions pharmaceutiques les refermant.**

(30) Priorité: **19.11.87 FR 8715980**

(43) Date de publication de la demande: **24.05.89 Bulletin 89/21**

(45) Mention de la délivrance du brevet: **12.01.94 Bulletin 94/02**

(84) Etats contractants désignés: **AT BE CH DE ES FR GB GR IT LI LU NL SE**

(56) Documents cités:
**FR-A- 2 381 048**
**FR-A- 2 590 572**
**GB-A- 2 107 317**

(73) Titulaire: **ROUSSEL-UCLAF**
**35, Boulevard des Invalides**
**F-75007 Paris(FR)**

(72) Inventeur: **Aktogu, Nurgün**
**2, rue Edmond About**
**F-92350 Le Plessis Robinson(FR)**
Inventeur: **Clémence, François**
**2, rue Turgot**
**F-75009 Paris(FR)**
Inventeur: **Oberlander, Claude**
**2, rue Paul Albert**
**F-75018 Paris(FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

L'invention concerne de nouveaux dérivés substitués de 20,21-dinoréburnaménine, leur procédé de préparation et les nouveaux intermédiaires ainsi obtenus, leur application comme médicaments et les compositions pharmaceutiques les renfermant.

Dans les brevets français FR 2 381 048 et britannique GB 2 107 317 sont décrits des produits comportant également un noyau du type dinoréburnaménine, ce sont des oxygénateurs et vasorégulateurs cérébraux.

L'invention a pour objet de nouveaux composés de formule (I) :

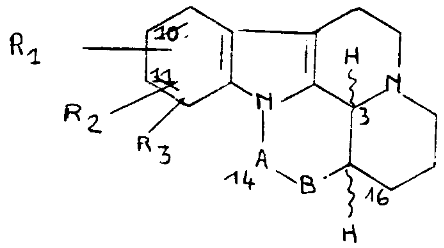

(I)

dans laquelle $R_1$, $R_2$ et $R_3$, identiques ou différents, représentent un atome d'hydrogène, un atome d'halogène, un radical alcoyle ou alcoxy renfermant de 1 à 5 atomes de carbone, un radical hydroxy, trifluorométhyle, nitro, amino, alcoylamino, dialcoylamino dans lequel le radical alcoyle renferme de 1 à 5 atomes de carbone, un radical acylamino dans lequel le radical acyle représente le reste d'un acide aliphatique renfermant jusqu'à 6 atomes de carbone, $R_1$, $R_2$ et $R_3$ ne pouvant pas représenter simultanément un atome d'hydrogène et dans laquelle le groupement :

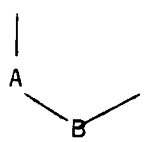

représente soit :

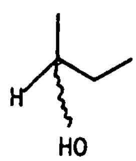

,

soit :

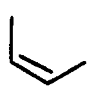

,

ou soit :

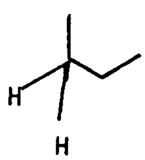

lesdits produits de formule (I) étant sous toutes les formes isomères possibles racémiques ou optiquement actives ainsi que les sels d'addition avec les acides minéraux ou organiques desdits produits de formule (I).

Dans les produits de formule (I), l'atome d'hydrogène en position 3 et l'atome d'hydrogène en position 16, peuvent chacun occuper l'une ou l'autre des orientations alpha et béta, ce qui détermine l'existence de diastéréoisomères cis et trans. De même, le radical hydroxy en position 14 peut être sous la forme alpha ou béta, lorsque le groupement :

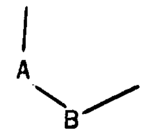

représente

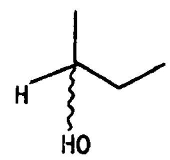

dans les produits de formule (I).

Lorsque $R_1$, $R_2$ et $R_3$ représentent un radical alcoyle, il s'agit de préférence des radicaux méthyle, éthyle, n-propyle ou isopropyle, mais ces substituants peuvent également représenter un radical n-butyle, isobutyle, n-pentyle.

Lorsque $R_1$, $R_2$ et $R_3$ représentent un radical alkoxy, il s'agit de préférence d'un radical méthoxy ou éthoxy, mais ils peuvent aussi représenter un radical propoxy, isopropoxy, butoxy linéaire, secondaire ou tertiaire.

Lorsque $R_1$, $R_2$ et $R_3$ représentent un atome d'halogène, il s'agit de préférence de l'atone de chlore, mais ils peuvent aussi représenter un atome de fluor, de brome ou d'iode.

Lorsque $R_1$, $R_2$ et $R_3$ représentent un radical alcoylamino, il s'agit de préférence du radical méthylamino, éthylamino ou propylamino.

Lorsque $R_1$, $R_2$ et $R_3$ représentent un radical dialcoylamino, il s'agit de préférence du radical diméthylamino ou diéthylamino.

Lorsque $R_1$, $R_2$ et $R_3$ représentent un radical acylamino, il s'agit de préférence du radical acétylamino, propionylamino, butyrylamino.

Les sels d'addition avec les acides minéraux ou organiques des produits de formule I peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques, tel que l'acide méthanesulfonique, l'acide éthanesulfonique, l'acide propanesulfonique, les acides alcoyldisulfoniques tels que l'acide méthanedisulfonique, l'acide alpha, béta-éthanedisulfonique, les acides arylmonosulfoniques, tel que l'acide benzènesulfonique et les acides aryldisulfoniques.

L'invention a notamment pour objet les composés de formule (I) caractérisés en ce que $R_1$, $R_2$ et $R_3$ identiques ou différents représentent un atome d'hydrogène ou un radical méthyle, éthyle, méthoxy ou éthoxy, un atome de chlore, un radical hydroxy, trifluorométhyle ou nitro, sous toutes les formes isomères possibles racémiques ou optiquement actives, ainsi que les sels d'addition avec les acides minéraux ou organiques.

L'invention a aussi notamment pour objet les composés de formule (I) caractérisés en ce que l'un des trois substituants $R_1$ ou $R_2$ ou $R_3$ représente en position 10 ou 11 un radical méthyle, éthyle, méthoxy ou éthoxy, un atome de chlore, un radical hydroxy, trifluorométhyle ou nitro, les deux autres substituants représentant un atone d'hydrogène, sous toutes les formes isomères possibles, racémiques ou optiquement actives, ainsi que les sels d'addition avec les acides minéraux ou organiques.

L'invention concerne aussi particulièrement les composés de formule (I) caractérisés en ce que deux des trois substituants $R_1$, $R_2$ ou $R_3$ représentent en position 9, 10 ou 11 un atome de chlore, un radical méthyle, éthyle, méthoxy ou éthoxy, le troisième substituant représentant un atome d'hydrogène ou en ce que $R_1$, $R_2$ et $R_3$ représentent tous trois dans ces positions un atome de chlore ou un radical méthyle,

éthyle, méthoxy ou éthoxy sous toutes les formes isomères possibles racémiques ou optiquement actives, ainsi que les sels d'addition avec les acides minéraux ou organiques.

L'invention a tout particulièrement pour objet les composés de formule (I) caractérisés en ce que l'atome d'hydrogène en position 3 et l'atome d'hydrogène en position 16 sont trans, sous toutes les formes isomères possibles racémiques ou optiquement actives ainsi que les sels d'addition avec les acides minéraux ou organiques et ceux
caractérisés en ce que le groupement

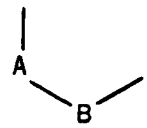

représente soit

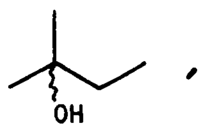

soit

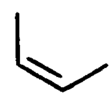

sous toutes les formes isomères possibles racémiques ou optiquement actives, ainsi que les sels d'addition avec les acides minéraux ou organiques.

Les composés de formule (I) préférés de l'invention sont ceux dont les noms suivent :

- [(+) (14alpha, 16alpha)] 14,15-dihydro 10-méthoxy 20,21-dinoréburnaménin-14-ol,
- [(±) (14alpha, 16alpha)] 14,15-dihydro 11-méthyl 20,21-dinoréburnaménin-14-ol,
- [(±) (16alpha)] 11-chloro 20,21-dinoréburnaménine,
- [(±) (16alpha)] 11-méthoxy 20,21-dinoréburnaménine,
- [(±) (16alpha)] 11-méthyl 20,21-dinoréburnaménine,

ainsi que leurs sels d'addition avec les acides minéraux ou organiques.

L'invention concerne aussi un procédé de préparation des composés de formule (I) caractérisé en ce que l'on réduit un composé de formule (II) :

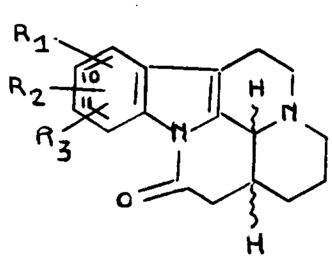

(II)

dans laquelle $R_1$, $R_2$ et $R_3$ ont les significations déjà indiquées pour obtenir un composé de formule ($I_A$) correspondant à un produit de formule (I) dans laquelle :

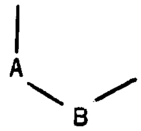

représente

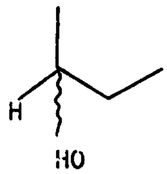

ledit composé de formule ($I_A$) étant, si désiré, déshydraté pour obtenir un composé correspondant de formule ($I_B$), représentant un composé de formule (I) dans laquelle :

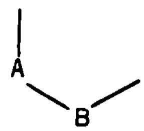

représente

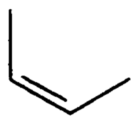

ledit composé de formule ($I_B$) étant, si désiré, réduit en composé correspondant de formule ($I_C$) représentant un composé de formule (I) dans laquelle :

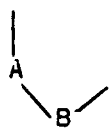

représente

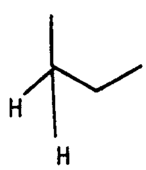

et traite si désiré tous les produits de formule (I) obtenus par un acide minéral ou organique pour en former le sel.

Dans des conditions préférentielles de mise en oeuvre de l'invention, le procédé ci-dessus est réalisé de la manière suivante :

- la réduction des composés de formule (II) en composé de formule ($I_A$) dans laquelle :

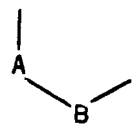

représente

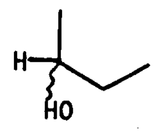

est réalisée avec un hydrure, notamment un hydrure mixte, tel que par exemple, l'hydrure mixte de lithium et d'aluminium, le diéthylhydrure de sodium et d'aluminium, l'hydroborure de sodium, l'hydroborure de lithium, l'hydrure de diisobutyl-aluminium.
- L'agent de déshydratation utilisé pour obtenir à partir des composés de formule ($I_A$) les composés de formule ($I_B$) dans laquelle :

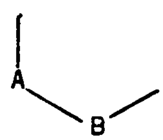

représente

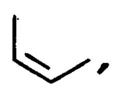

est un acide tel que l'acide chlorhydrique, l'acide bromhydrique, l'acide sulfurique, l'acide acétique, l'acide paratoluène sulfonique, l'acide méthanesulfonique ou un dérivé de cet acide tel que le trifluorométhane sulfonate de cuivre utilisé en quantité catalytique.

Le produit de formule ($I_B$) dans laquelle $R_1$, $R_2$ ou $R_3$ représente un radical acylamino peut être soumis, le cas échéant, à une hydrolyse basique pour éliminer le groupement acyle.

La base que l'on utilise de préférence est une base minérale telle que l'hydroxyde de sodium ou de potassium.

- L'agent de réduction auxquels sont soumis les composés de formule ($I_B$) pour obtenir les composés de formule ($I_C$) dans laquelle

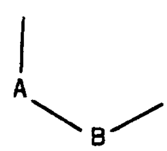

représente

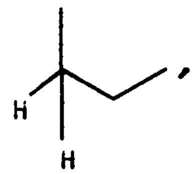

est l'hydrogène en présense d'un catalyseur tel que le platine ou le palladium.

- Les produits de formule (I) pour lesquels l'atome d'hydrogène en position 3 et l'atome d'hydrogène en position 16 sont en position cis ou trans, sont obtenus à partir des produits de formule (II) ayant la forme stéréochimique correspondante.
- On obtient le produit de formule (I) dans laquelle

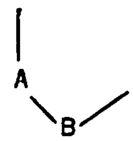

représente

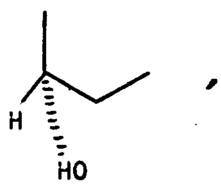

OH étant en position axiale par épimérisation en milieu acide du produit de formule (I) dans laquelle :

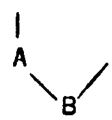

représente

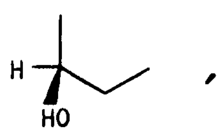

OH étant en position équatoriale.

- Les formes optiquement actives des produits de formule (I) peuvent être préparées par dédoublement des racémiques selon les méthodes usuelles, soit en dédoublant le produit de formule (II) racémique pour obtenir un produit de formule (II') 3alpha et un produit de formule (II") 16alpha que l'on soumet aux réactions de réduction et de déshydratation indiquée ci-dessus pour obtenir les produits de formule ($I'_A$), ($I'_B$), ($I'_C$) correspondant respectivement aux produits ($I_A$), ($I_B$), ($I_C$) 3alpha et les produits ($I_A$), ($I_B$), ($I_C$) correspondant respectivement aux produits ($I_A$), ($I_B$), ($I_C$) 16alpha, soit en dédoublant directement les produits de formule (I) racémiques.
- On utilise de préférence un composé optiquement actif tel que l'acide (+) (-) di-OO'-pivaloyl D ou L tartrique.

Les composés de formule (I) tels que définis ci-dessus ainsi que leurs sels d'addition avec les acides présentent d'intéressantes propriétés pharmacologiques.

Certains produits présentent notamment une affinité pour les récepteurs alpha 2 adrénergique.

Certains produits peuvent aussi présenter d'intéressantes propriétés nootropes (effet anti-amnésiant et réversion d'un déficit mnésique après lésion d'un cholinergique septale), anti-dépressives, protectrices neuronales, anti-anoxiques, anti-ischémiques.

Les propriétés justifient leur application en thérapeutique et l'invention également pour objet à titre de médicaments, les produits tels que définis par la formule (I) ci-dessus, lesdits produits de formule (I) étant sous toutes les formes isomères possibles racémiques ou optiquement actives, ainsi que les sels d'addition avec les acides minéraux ou organiques pharmaceutiquement acceptables desdits produits de formule (I).

L'invention a plus particulièrement pour objet, à titre de médicament, les produits (I) suivants :

- [(±) (14alpha, 16alpha)] 14,15-dihydro 10-méthoxy 20,21-dinoréburnaménin-14-ol,
- [(±) (14alpha, 16alpha)] 14,15-dihydro 11-méthyl 20,21-dinoréburnaménin-14-ol,
- [(±) 16alpha] 11-chloro 20,21-dinoréburnaménine,
- [(±) 16alpha] 11-méthoxy 20,21-dinoréburnaménine,
- [(±) 16alpha] 11-méthyl 20,21-dinoréburnaménine,

ainsi que leurs sels d'addition avec les acides minéraux ou organiques pharmaceutiquement acceptables.

Les médicaments, objet de l'invention, peuvent être utilisés dans le traitement des insuffisances cérébrales d'origine anoxique ou ischémique dans les troubles de la mémoire et de l'attention. Ils peuvent également être utilisés comme anti-dépresseurs.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine, comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 10 à 200 mg parjour chez l'adulte, par voie orale.

L'invention a également pour objet à titre de produits chimiques nouveaux, nécessaires à la préparation des produits de formule (I), les produits de formule (II) à l'exception des produits de formule ($II_A$) :

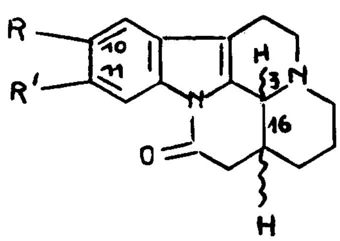

($II_A$)

pour lesquels R et R' identiques ou différents représentent un radical hydroxy ou méthoxy.

Les produits de formule ($II_A$) correspondent à certains produits de formule (I) du brevet belge n¤ 764166.

Les produits de formule (II) peuvent présenter certaines des propriétés pharmacologiques indiquées précédemment pour les produits de formule (I).

Les produits de formule (II) sont préparés suivant le procédé donné dans le brevet français n¤ 2168853 pour la déséthyléburnamonine à partir du 2,3,4,6,7,12-hexahydro indolo(2,3-a)quinolizine de formule :

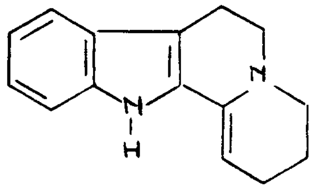

les dérivés substitués de la 20,21-dinoréburnaménine de la présente invention sont donc préparés à partir des dérivés substitués correspondant du 2,3,4,6,7,12-hexahydro indolo(2,3-a) quinolizine.

Un autre procédé de préparation des produits de formule (II) consiste à soumettre un produit de formule

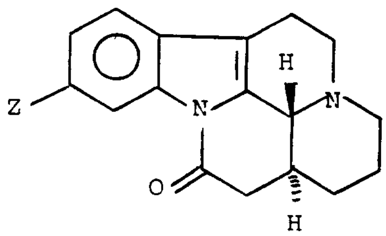

dans laquelle Z représente un atome d'hydrogène à une réaction de nitration pour obtenir un produit de formule ($II'_A$) :

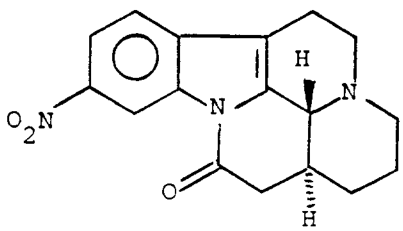

($II'_A$)

que l'on réduit le cas échéant pour obtenir un produit de formule ($II_B$) :

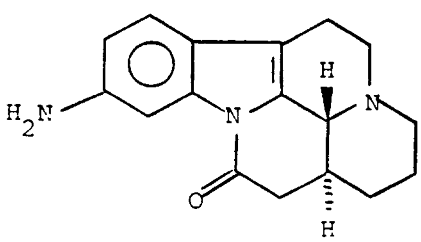

($II_B$)

que le cas échéant, soit l'on soumet à une réaction d'alkylation ou d'acylation, soit l'on transforme en sel de diazonium à partir duquel on prépare par les procédés connus les dérivés de formule ($II_C$) :

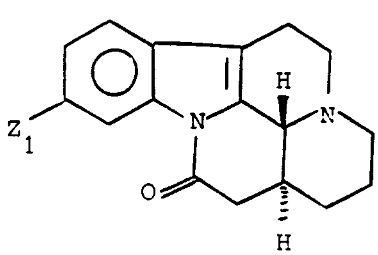

($II_C$)

dans laquelle $Z_1$ représente un radical hydroxy ou un atome d'halogène que l'on transforme le cas échéant en dérivés correspondants dans lesquels $Z_1$ représente un radical alkoxyle ou alkyle.

Des exemples correspondants de préparation de produit de formule (II) sont donnés ci-après dans la partie expérimentale.

Les exemples donnés ci-après illustrent l'invention.

**Exemple 1 : [(±) (14alpha, 16alpha)] 11-chloro 14,15-dihydro 20,21-dinoréburnaménin-14-ol**

On refroidit à -10¤C une solution de 4,6 g de (±) (16alpha) 11-chloro 20,21-dinoréburnaménin-14(15H)-one dans 50 cm3 de toluène, ajoute lentement 12 cm3 d'une solution 2,36 M de dihydrure de diéthyl-aluminium-sodium dans le toluène en maintenant la température du milieu aux environs de -5¤C. On agite à 0¤C pendant 30 minutes, ajoute de l'eau pour détruire l'excès de réducteur, puis 100 cm3 d'eau et agite la suspension obtenue pendant 16 heures. On essore, lave abondamment à l'eau jusqu'à obtention d'un pH neutre des eaux de lavage, sèche à 100¤C sous pression réduite, dissout dans un mélange chlorure de méthylène-méthanol (2-1) filtre et amène à sec. On empâte le résidu avec un mélange méthanol-soude 5N (100 cm3-30 cm3) et porte au reflux pendant 15 heures. On essore le précipité, lave à l'eau, empâte au méthanol au reflux plusieurs fois, sèche et obtient 2,5 g de produit attendu F = 257¤C.

Spectre RMN (DMSO 250 MHz ppm) :
isomère avec OH axial
5,90 : >C(H)OH
trace d'isomère avec OH équatorial ( 2%)
5,53 : >C(H)OH

**Exemple 2 : [(±) (16alpha)] 11-chloro 20,21-dinoréburnaménine et son fumarate neutre.**

On porte au reflux pendant 15 heures 1,9 g de [(±) (14alpha, 16alpha) 11-chloro 14,15-dihydro 20,21-dinoréburnaménin-14-ol, 40 mg d'acide paratoluène sulfonique dans 40 cm3 de toluène. On filtre, lave l'insoluble au chlorure de méthylène et concentre à sec le filtrat. On chromatographie le résidu sur silice, élue par un mélange acétate d'éthyle-chlorure de méthylène (1-1) et obtient 1,2 g de produit attendu. F = 118¤C.

On dissout 1,16 g de ce dernier dans 50 cm3 d'éthanol anhydre, ajoute 472 mg d'acide fumarique et maintient sous agitation pendant 3 heures. On essore, recristallise dans l'éthanol et obtient 1,06 g de fumarate neutre. F = 215¤C.

Spectre RMN ($CDCl_3$ 250 MHz ppm) :

(base)

5,11 (dd) J = 2 et 7,5 : H éthylénique en béta de l'indole
6,88 (dd) J = 3 et 7,5 : H éthylénique en alpha de l'indole
7,05 (dd) J = 2 et 8,5) : $H_5$
7,30 (d,J=2) : $H_7$ ) indole
7,34 (d,J=8,5) : $H_4$
1,3 à 3,2 les autres protons

**Exemple 3 :[(±) (14béta)] 10-chloro 14,15-dihydro 20,21-dinoréburnaménin 14-ol**

On refroidit à 0¤C 5,5 g de (±) 10-chloro 20-21-dinoréburnaménin-14(15H)-one dans 50 cm3 de toluène et ajoute goutte à goutte 14 cm3 d'une solution de dihydrure de diéthyl-aluminium-sodium et agite 1 heure à 0¤C. On ajoute alors très lentement à 0¤C 50 cm3 d'eau et poursuit l'agitation pendant 1 heure de la suspension obtenue. On essore, lave abondamment à l'eau et obtient 4,73 g de produit brut F = 244¤C.

On recristallise 2,4 g de ce dernier dans 50 cm3 de tétrahydrofuranne, refroidit à -20¤C, essore et sèche sous pression réduite et obtient 1,7 g de produit F = 251¤C.

On reprend ce produit par un mélange chlorure de méthylène-méthanol, filtre, amène à sec, lave le solide obtenu par 50 cm3 de méthanol. Après séchage à 70¤C sous pression réduite, on obtient 1,42 g de produit (OH équatorial) F = 252¤C.

Spectre RMN (DMSO 250 MHz ppm) :
Produit brut = mélange contenant 10 à 15 % d'OH axial pour 85 à 90 % d'OH équatorial

5,54 (dt) : >(CH) -OH-axial
5,90 (m) : >(CH) -OH équatorial
4,17 et 4,11 : >(CH) -N jonction cis
6,48 (d) : OH
7,06 (dd) : $H_6$ indole
7,41 (d) : $H_4$ "
7,66 (d) :
7,50 (d) : $H_7$ "

0,63 (m) : 1H
1,3 à 3,2 : les autres protons

**Exemple 4 : [(±) (14alpha, 16alpha)] 10-chloro 14,15-dihydro 20-21-dinoréburnaménine-14-ol**

On dissout 7,5 g de [(±) (16alpha)] 10-chloro 20,21-dinoréburnaménin-14(15H)-one dans 100 cm3 de toluène et ajoute goutte à goutte à 0¤C 19,5 cm3 d'une solution (1,8 M) de dihydrure de diéthyl-aluminium-sodium dans le toluène, puis maintient sous agitation à 0¤C pendant 1 heure. On ajoute alors de l'eau, très lentement sans dépasser 5¤C et maintient l'agitation pendant 1/4 heure. On essore le précipité, le lave abondamment à l'eau, sèche sous pression réduite à 70¤C et obtient 7,46 g de produit brut correspondant à l'isomère avec OH équatorial. On met en suspension 3 g de ce dernier dans 30 cm3 d'acide chlorhydrique N et agite pendant 15 heures à température ambiante. On amène le pH à 10 par addition de soude, essore, lave à l'eau et obtient 2,8 g de produit. Après deux recristallisations dans le tétrahydrofuranne, on obtient 760 mg de produit attendu. On concentre à sec les eaux-mères, traite le résidu par l'acide chlorhydrique N, alcalinise et recristallise comme précédemment et récupère 1,03 g du produit attendu.

Spectre RMN (DMSO, 250 MHz, ppm) :
OH équatorial
5,89 (m) : >C(H)OH équatorial
6,27 (d, J = 7) : OH-axial
7,05 (d, d, J = 2 et 8,5) : $H_6$-indole
7,39 (d, J = 2) : $H_4$-indole
7,44 (d, J = 8,5) : $H_7$-indole
1,1 à 3,1 : les autres protons

**Exemple 5 : Hémifumarate de [(±) 16alpha] 10-chloro 20,21-dinor éburnaménine**

On prépare une suspension avec 6 g de [(±) (14alpha, 16alpha)] 10-chloro 14,15-dihydro 20,21-dinoréburnaménin-14-ol dans 100 cm3 de toluène, ajoute en une fois 300 mg d'acide paratoluène-sulfonique et porte au reflux pendant 10 heures. Après refroidissement à température ambiante, on essore l'insoluble, le lave à l'acétate d'éthyle et amène à sec le filtrat. On empâte le résidu dans 50 cm3 d'une solution de bicarbonate de sodium, essore, lave abondamment à l'eau, sèche sous pression réduite, chromatographie sur silice en éluant par un mélange chlorure de méthylène-méthanol (97-3) et isole 3,9 g de produit sous forme de base. F = 151¤C.

On dissout 1,5 g de ce dernier dans un mélange acétate d'éthyle-isopropanol (8-2), ajoute 305 mg d'acide fumarique et maintient l'agitation pendant 1 heure 30 minutes à température ambiante. On essore, lave successivement par 50 cm3 d'acétate d'éthyle et 25 cm3 d'isopropanol, sèche sous pression réduite à 70¤C et obtient 1,57 g F = 241¤C.

Spectre RMN ($CDCl_3$250 MHz ppm) :
5,10 (d,d) J = 2 et 7,5 : H éthyléniques en béta de N
6,89 (d,d) J = 3 et 7,5 : H éthyléniques en alpha de N
7,09 (d,d) J = 2 et 8,5 : $H_6$ indole
7,2 (d, J = 8,5) : $H_7$ indole
7,41 (d, J = 2) : $H_4$ indole
1,3 à 3,2 les autres protons.

**Exemple 6 : hémifumarate de (±) 10-chloro 20,21-dinoréburnaménine**

On dissout 1,6 g de [(±)(14béta)] 10-chloro 14,15-dihydro 20,21-dinoréburnaménin 14-ol dans 20 cm3 d'acide acétique et porte au reflux pendant 2 heures. On concentre la solution, ajoute de l'eau et alcalinise à pH 10 avec de la soude concentrée. On extrait au chlorure de méthylène le précipité obtenu, lave la phase organique à l'eau, sèche, élimine les solvants, empâte le résidu plusieurs fois dans 50 cm3 de xylène, puis concentre sous pression réduite à 80¤C. On chromatographie sur silice le résidu en éluant par un mélange chlorure de méthylène-méthanol 95-5 et isole 1,01 g de produit sous forme de base. On dissout 1 g de ce dernier dans un mélange acétate d'éthyle-isopropanol (20-10), ajoute 204 mg d'acide fumarique, agite pendant 48 heures à température ambiante. On essore, lave par 10 cm3 d'isopropanol, puis 20 cm3 d'acétate d'éthyle. Après séchage sous pression réduite à 70¤C, on obtient 750 mg de produit attendu. F = 188¤C.
Spectre RMN (DMSO 250 MHz ppm) :
4,53 (m) : CH - N jonction cis
5,41 (d,d, Jr 6 et 8) : CH = CH-N
7,31 (d Jr8) : N - CH = CH
7,14 (d,d, J = 2,5 et 9) : $H_6$ indole
7,48 (d, J = 2,5) : $H_4$ indole
7,61 (d, J 9) : $H_7$ indole
0,6 à 3,3 : les autres protons
6,6 (s) : H éthyléniques de l'acide fumarique

**Exemple 7 : [(±) (14béta, 16alpha)] 14,15-dihydro 11-méthyl 20,21-dinor éburnaménin-14-ol**

On opère comme à l'exemple 1 à partir de 2,1 g de [(±) 16alpha)] 11-méthyl 20,21-dinoréburnaménin-14(15H)-one. Après introduction du réactif, on maintient l'agitation à 0¤C pendant 1 heure, puis on ajoute 50 cm3 d'eau très lentement et agite encore 1 heure à 0¤C. On essore le précipité, lave abondamment à l'eau, sèche à 80¤C sous pression réduite et obtient 1,7 g de produit que l'on dissout dans un mélange chlorure de méthylène-méthanol (2-1), filtre, amène à sec le filtrat. On empâte le résidu dans 200 cm3 de méthanol, sèche et obtient 1,1 g de produit. F ≃ 255¤C.
Spectre RMN (DMSO 250 MHz ppm) :
OH équatorial possible
2,38 (s) : $CH_3$-O
5,46 (dt) (J = 9 et 5,5) : C H -OH axial
6,37 (d) (J = 9) : OH équatorial
6,84 (d,d) (J = 8 et 1) : $H_5$ indole
7,24 (d, J = 8) : $H_4$ indole
7,46 (S large) : $H_7$ indole
1,1 à 3,0 : les autres protons

**Exemple 8 : [(±) (14alpha, 16alpha)] 14,15-dihydro 11-méthyl 20,21-dinoréburnaménin-14-ol**

On met en suspension 3,8 g de [(±)(14béta, 16alpha)] 14,15-dihydro 11-méthyl 20,21-dinoréburnaménin 14-ol dans 100 cm3 d'acide chlorhydrique 0,5 N, sous agitation, sous atmosphère inerte pendant 24 heures. On ajoute 800 cm3 d'eau à 40¤C pour solubiliser le chlorhydrate, alcalinise par addition d'ammoniaque à 20%. Après 1/4 d'heure d'agitation, on essore le précipité, lave abondamment à l'eau jusqu'à pH neutre des eaux de lavage, sèche sous pression réduite à 70¤C et obtient 3,3 g de produit attendu. F = 232¤C.
Spectre RMN (DMSO 250 MHz ppm) :
2,39 (s) : $CH_3$Ø
5,85 : >CⒽOH équatorial
6,83 : $H_5$ indole
7,23 : $H_4$ et $H_7$ indole
6,08 : OH axial

**Exemple 9 : [(±) (14béta)] 14, 15-dihydro 11-méthyl 20,21-dinoréburménin-14-ol**

On opère comme à l'exemple 1 à partir de 9,7 g de (±) 11-méthyl 20,21-dinoréburnaménin-14(15H)-one, agite encore 1 heure après l'introduction du réactif et ajoute à 0¤C très lentement de l'eau pour

détruire l'excès de réducteur, puis 100 cm3 d'eau et agite pendant 3 heures à température ambiante. On essore, lave abondamment le précipité jusqu'à pH neutre des eaux de lavage et obtient 8,11 g de produit brut. On dissout 3 g de ce dernier dans 100 cm3 d'un mélange chlorure de méthylène-méthanol (2-1), filtre et amène à sec le filtrat. On recristallise le résidu dans 60 cm3 d'acétate d'éthyle, essore, sèche sous pression réduite à 70¤C et obtient 1,63 g de produit attendu F = 200¤C.
Spectre RMN (DMSO 250 MHz ppm) :

OH équatorial
4,15 (d) : -CH-N

5,49 (m) : -C Ⓗ -OH axial

6,29 (d) : OH équatorial

6,84 (d) : $H_5$
7,25 (d) : $H_4$ indole
7,48 : $H_7$

2,39 (s) : = CMe

1,16 à 3,14 (m) : autres protons

**Exemple 10 : Fumarate de [(±) 16alpha)] 11-méthyl 20,21-dinoréburnaménine**

On opère comme à l'exemple 2 à partir de 6,8 g de [(±) (14béta, 16alpha)] (±) 14,15-dihydro 11-méthyl 20,21-dinoréburnaménin-14-ol en portant au reflux 8 heures et en éluant par un mélange chlorure de méthylène-méthanol (95-5). On isole 4,8 g de produit sous forme de base F = 130¤C. On dissout 1,2 g de ce dernier dans un mélange isopropanol-acétate d'éthyle (50 cm3-50 cm3), ajoute 527 mg d'acide fumarique et agite 16 heures à température ambiante. On essore le précipité, lave avec 40 cm3 d'isopropanol, sèche à 80¤C sous pression réduite et obtient 1,38 g de produit attendu. F > 260¤C.
Spectre RMN ($CDCl_3$ 250 MHz ppm) :
(base)
structure possible avec jonction trans
1,41 à 3,17 : les $CH_2$ et CH
5,05 (d,d J = 2 et 7) : C Ⓗ = CH-N
6,91 à 6,96 : $H_5$ et l'autre = CH
7,13 : $H_7$ indole
7,34 : $H_5$ indole

**Exemple 11 : Fumarate neutre de (±) 11-méthyl 20,21-dinoréburnaménine**

On opère comme à l'exemple 2 à partir de 5,33 g de [(±) (14béta)] 14,15-dihydro 11-méthyl 20,21-dinoréburnamémin-14-ol en éluant par un mélange : chlorure de méthylène-méthanol (93-7), on isole 5 g de produit sous forme de base F = 66¤C. On dissout 2 g de ce dernier dans 100 cm3 d'un mélange isopropanol-acétate d'éthyle (6-4), on ajoute 439 mg d'acide fumarique, agite 16 heures, essore, lave avec 40 cm3 d'éthanol et obtient 1,89 g de produit attendu F = 250¤C.
Spectre RMN ($CDCl_3$ 250 MHz ppm) :

(base)
jonction cis

2,47 (s) : $CH_3-\phi$
4,53 (m) : >CH-N jonction cis
5,29 (dd J = 6 et 8) : CH = CH-N
6,92 (d J = 8) : N- CH = C
6,94 (dd) J = 8 et 1 : $H_5$ } indole
7,13 (s) : $H_7$
7,35 (d J = 8) : $H_4$

0,8 à 3,4 les autre protons

**Exemple 12 : Fumarate neutre de [(±) (16alpha)] 14,15-dihydro 11-méthyl 20,21-dinoréburnaménine**

On hydrogène 2,9 g [(±) (16alpha)] 11-méthyl 20,21-dinoréburnaménine dans 90 cm3 d'éthanol en présence d'oxyde de platine pendant 2 heures. On filtre, amène à sec le filtrat et obtient 3 g de produit sous forme de base. F = 143¤C. On opère comme à l'exemple 2 à partir de 2,87 g de ce dernier pour obtenir 2,83 g de fumarate attendu. F = 235¤C.
Spectre RMN ($CDCl_3$ 250 MHz ppm) :

(base)

2,46 : $CH_3$-
3,65 (J = 11, 11 et 5) : } $CH_2$ sur N indole
4,18 (J = 11-5.1) :
6,91 : 1H } aromatiques
7,03 : 1H
7,34 : 1H

**Exemple 13 : [(±) (14béta, 16alpha)] 14,15-dihydro 10-méthyl 20,21-dinoréburnaménin-14-ol**

On opère comme à l'exemple 3 à partir de 2,6 g de [(±) (16alpha)] 10-méthyl 20-21-dinoréburnaménin 14(15H)one et obtient 2,7 g de produit brut. On reprend celui-ci par un mélange chlorure de méthylène méthanol (2-1), filtre, concentre à sec le filtrat, lave le résidu au méthanol et obtient 2 g de produit attendu. F =>260¤C.
Spectre RMN (DMSO 250 MHz ppm) :

jonction trans avec OH équatorial

2,36 (s) : $CH_3$

5,45 (dt, J = 5,5-9,9) : >CH OH axial
6,4 (d J = 9) : OH équatorial

7,15 (s) : $H_4$ indole

6,88 (d) : $H_6$ indole
7,52 (d, J 8) : $H_7$

1,1 à 3,0 les autres protons.

**Exemple 14 : [(±) (14béta)] 14,15-dihydro 10-méthyl 20,21-dinoréburnaménin-14-ol**

On opère comme à l'exemple 3 à partir de 7,9 g (±) 10-méthyl 20,21-dinoréburnaménin 14(15H)-one et obtient 6,73 g de produit brut. On dissout 3 g de ce dernier dans du chlorure de méthylène, filtre, amène à sec le filtrat et recristallise le résidu dans 65 cm3 d'acétate d'éthyle. On obtient 2,13 g de produit attendu. F = 187¤C.
Spectre RMN (DMSO 250 MHz ppm) :

jonction cis, OH équatorial
2,36 : $CH_3$
4,15 : >CH-N
5,47 : - C(H) - OH axial

**Exemple 15 : [(±) (14alpha 16alpha)]14,15-dihydro 10-méthyl 20,21-dinoréburnaménin-14-ol**

On opère comme à l'exemple 8 à partir de 2,2 de [(±) (14béta, 16alpha)] 14,15-dihydro 10-méthyl 20,21-dinoréburnaménin-14-ol, obtient 2 g de produit brut que l'on recristallise dans 150 cm3 de tétrahydrofuranne pour isoler 850 mg de produit avec OH axial. Des eaux mères retraités, on recueille encore 455 mg de produit.
Spectre RMN DMSO 250 MHz ppm :

2,36 (s) : $CH_3$-ø
5,86 (m) : >C(H)- OH équatorial
6,10 (d, J = 6,5) : OH axial

6,88 (d) : $H_6$ )
7,13 (s) : $H_4$ ) indole
7,31 (d, J = 8) : $H_7$ )

1,1 à 3,05 les autres protons

**Exemple 16 : Hémifumarate de [(±) (16alpha)] 10-méthyl 20,21-dinoréburnaménine**

On opère comme à l'exemple 2 à partir de 4,4 g de [(±) (14béta, 16alpha)] 14,15-dihydro 10-méthyl 20,21-dinoréburnaménin-14-ol, on chromatographie le résidu sur silice en éluant par un mélange chlorure de méthylène-méthanol (97-3) et obtient 3 g de produit sous forme de base. F = 154¤C. On dissout 1,4 g de celui-ci dans 80 cm3 d'un mélange isopropanol-acétate d'éthyle (1-1), on ajoute 307 mg d'acide fumarique et agite pendant 4 heures à température ambiante. On essore, lave à l'acétate d'éthyle, puis à l'isopropanol, sèche sous pression réduite à 80¤C et obtient 1,3 g de produit attendu F = 210¤C.
Spectre RMN ($CDCl_3$ 250 MHz ppm) :

jonction trans

1,42 à 3,18 : les $CH_2$ et CH

2,44 : = C-Me

5,05 (d,d J = 2 et 7) : CH = CH-N

6,94 et 6,98 ) : aromatiques et l'autre CH
7,19 à 7,26 )

**Exemple 17 : Fumarate de (±) 10-méthyl 20,21-dinoréburnaménine**

On opère comme à l'exemple 16 à partir de 4,1 g de (±) 14alpha 14,15-dihydro 10-méthyl 20,21-dinoréburnaménin-14-ol et obtient 3,6 de produit huileux. On dissout 1,508 g de ce dernier dans 50 cm3 d'isopropanol, ajoute 331 mg d'acide fumarique et agite 5 heures à température ambiante. On essore, lave à l'isopropanol puis à l'éther isopropylique. On obtient 1,16 g de produit attendu. F = 180¤C.

Spectre RMN ($CDCl_3$ 250 MHz ppm) :

base

2,44 (s) : $CH_3$-Ø

4,53 (m) : >C (H)- N jonction cis

5,28 (dd J = 6,5 et 7,5) : -(CH)= CH-N

6,91 (d, J = 7,5) : N -(CH)= CH

7,00 (d J = 8) : $H_6$ )
7,21 (d J 8) : $H_7$ ) indole
7,26 (s) : $H_4$ )

0,9 (m) : 1H

1,44 à 3,4 : les autres protons

**Exemple 18 : Hémifumarate de [(±) (16alpha)] 14,15-dihydro 10-méthyl 20,21-dinoréburnaménine**

On opère comme à l'exemple 12 à partir de 1,6 g de [(±) (16alpha)] 10-méthyl 20-21-dinoréburnaménine et obtient 1,4 g de produit attendu F = 146¤C sous forme de base. A partir de 1,369 g de celui-ci et 298 mg d'acide fumarique, on obtient après recristallisation dans l'éthanol 0,84 g de fumarate attendu F = 254¤C.

Spectre RMN ($CDCl_3$ 250 MHz ppm) :

jonction trans

2,43 : $CH_3$ - ø

3,65 (dt) :
4,18 (m) : $CH_2$ -N-indole

6,95 (d,d) : $H_6$
7,13 (d) : $H_7$ indole
7,25 : $H_4$

1,22 (m) : 1H

1,5 à 3,2 : 13H les autres protons

**Exemple 19 : Hémifumarate de (±) 14,15-dihydro, 10-méthyl 20,21-dinoréburnaménine**

On opère comme à l'exemple 12 à partir de 2 g de (±) 10-méthyl 20,21-dinoréburnaménine et obtient 1,8 g de produit sous forme de base F = 138¤C. On salifie 1,6 g de ce dernier dans 100 cm3 d'acétate d'éthyle et 50 cm3 d'isopranol avec 697 mg d'acide fumarique pour obtenir, après cristallisation dans l'isopropanol 1,55 g de produit attendu F = 210¤C.
Spectre RMN ($CDCl_3$ 250 MHz) ppm :

fonction cis

2,46 (s) : $CH_3$-ø

3,73 (dt J 5-11,5-12) :
4,08 (d,d J = 6-11,5 : $CH_2$-N-indole

4,33 (m) :>C H- N jonction cis

7,0 (d) : $H_6$
7,17 (d) : $H_7$ indole
7,28 (s) : $H_4$

**Exemple 20 : [(±) 14béta] 14,15-dihydro 11-méthoxy 20,21-dinoréburnaminin-14-ol**

On opère comme à l'exemple 3 à partir de 3,5 g de (±) 11-méthoxy 20,21-dinoréburnaménin-14(15H)-one et obtient 8 g d'isomère avec OH équatorial. F = 230¤C. On recristallise 3,5 g du produit brut dans un mélange acétate d'éthyle-isopropanol (1-1) et recueille 2 g de produit attendu F = 230¤C.
Spectre RMN (DMSO 250 MHz ppm) :

OH équatorial

3,76 (s) : $OCH_3$

4,13 ≃ : >C Ⓗ - N équatorial

5,49 (dt J 5-9 et 9) : >C Ⓗ - OH axial

6,67 (dd J = 2 et 8,5) : $H_5$ ) indole

7,25 (m) : $H_4$ et $H_7$

0,63 : 1H

1,2 à 3,2 : les autres protons

**Exemple 21 : [(±) (14béta 16alpha)] 14,15-dihydro 11-méthoxy 20,21-dinoréburnaménin-14-ol**

On opère comme à l'exemple 3 à partir de 8 g de [(±) 16alpha], 11-méthoxy 20,21-dinoréburnaménin-14(15H)-one et obtient 7,91 g de produit brut F = 250¤C. On empâte 1,5 g de celui-ci dans 15 cm3 de soude 5N et 15 cm3 de méthanol, porte au reflux pendant 3 heures. Après refroidissement, on essore le précipité, lave au méthanol puis abondamment à l'eau. On obtient 11 g de produit attendu.

Spectre RMN (DMSO 250 MHz ppm) :

OH équatoral

3,75 (s) : $OCH_3$

5,46 (dt, J = 5,5 et 9) : >C Ⓗ - OH axial

6,47 (d, J = 9) : OH équatorial

6,66 (dd, J = 2 et 8,5) : $H_5$ )

7,21 (d, J = 2) : $H_7$ ) indole

7,23 (d, J = 8,5) : $H_4$ )

1,05 à 3,02 les autres protons

**Exemple 22 : [(±) (14alpha, 16alpha)] 14,15-dihydro 11-méthoxy 20-21-dinoréburnaménin 14-ol**

On agite pendant 72 heures 5 g de [(±) 14béta, 16alpha] 14,15-dihydro 11-méthoxy 20,21-dinoréburnaménin-14-ol dans 100 cm3 d'acide chlorhydrique N, on amène le pH de la suspension à 7 par addition de soude 2N. On essore le précipité, lave abondamment à l'eau, sèche et sous pression réduite à 70¤C obtient 4,4 g de produit brut. On recristallise dans le tétrahydrofuranne et obtient 2,16 g de produit attendu F = 245¤C.

Spectre RMN (DMSO 250 MHz ppm) :

OH axial

3,77 (s) : $OCH_3$

5,85 (m) : >C Ⓗ - OH équatorial

6,18 (d, J = 6,5) : OH axial

6,64 (d, d, J = 2 et 8,5) : $H_5$

7,01 (d,J = 2) : $H_7$ indole

7,21 (d,J = 8,5) : $H_4$

1,05 à 3,05 les autres protons.

**Exemple 23 : Hemifumarate de (±) 11-méthoxy 20-21-dinoréburnaménine**

On opère comme à l'exemple 2 à partir de 1,15 g de [(±) (14-béta)] 14,15-dihydro 11-méthoxy 20,21-dinoréburnaménin-14-ol. On amène à sec la solution, reprend le résidu par le chlorure de méthylène, lave avec de la soude 0,1 N, puis à l'eau, sèche puis amène à sec la phase organique. Après chromatographie du résidu sur silice, en éluant par un mélange acétate d'éthyle-triéthylamine (95-5), on obtient 750 mg de produit sous forme de base. F = 105¤C. On dissout 1,208 g de base dans 40 cm3 d'un mélange isopropanol-acétate d'éthyle (1-1) ajoute 250 mg d'acide fumarique, agite 16 heures à température ambiante, essore, lave à l'isopropanol et à l'acétate d'éthyle et obtient 1,107 g. F = 225¤C.
Spectre RMN ($CDCl_3$ 250 MHz ppm) :

jonction cis

3,86 (s) : $OCH_3$

4,51 (m) : >CH-N fonction cis

5,29 (d,d J = 6 et 7,5

6,89 (d, J = 7,5) : éthyléniques

6,76 (d,d J = 2 et 8,5) : $H_5$

6,84 (d, J = 2) : $H_7$ indole

7,34 (d, J = 8,5) : $H_4$

0,90 (m) : 1H

1,45 à 3,40 : les autres protons

**Exemple 24 : Hemifumarate de [(±) 16alpha] 11-méthoxy 20,21-dinoréburnaménine**

On opère comme à l'exemple 23 à partir de 2,2 de [(±) (14béta, 16alpha)] 14,15-dihydro 11-méthoxy 20,21-dinoréburnaménin 14-ol et obtient après chromatographie sur silice,(éluant : chlorure de méthylène-acétate d'éthyle 1-1) 1,27 g de produit sous forme de base F = 128¤C. On prépare le fumarate à partir de 1,27 g de base et obtient 1,23 g de produit attendu. F = 193¤C.
Spectre RMN (DMSO 250 MHz ppm) :

3,78 (s) : $OCH_3$

5,09 (dd, J = 2 et 8) : H éthylénique en béta de N

6,68 (dd, J = 2 et 8) : H éthylénique en alpha de N

7,19 (d,J = 2) : $H_7$ ) indole

7,25 à 7,35 : $H_5$ et $H_4$ ) indole

6,61 H éthylénique de l'acide fumarique

1,2 à 3,15 : les autres protons

**Exemple 25 : Fumarate neutre de [(±) (16alpha)] 14,15-dihydro 11-méthoxy 20,21-dinoréburnaménine**

On opère comme à l'exemple 12 à partir de 2,23 g de [(±) (16alpha)] 11-méthoxy 20,21-dinoréburnaménine et 220 mg de palladium sur charbon à 10% et obtient 2,2 g de produit huileux. On empâte à l'éther isopropylique, essore, sèche sous pression réduite à 50¤C et obtient 1,17 g de produit sous forme de base F = 126¤C. Des liqueurs mères, on récupère 740 mg de produit. On prépare le fumarate neutre à partir de 1,17 g de base dissous dans 20 cm3 d'isopropanol et 30 cm3 d'acétate d'éthyle. On obtient 1 g de produit attendu F = 216¤C.
Spectre RMN ($CDCl_3$ 90 MHz) base :

3,88 ppm : $OCH_3$

6,72 à 6,83 : $H_5$ et $H_7$ ) indole

7,31 " 7,42 : $H_4$ ) indole

1,24 à 4,28 : autres protons

**Exemple 26 : [(±) 14béta] 14,15-dihydro 10-méthoxy 20,21-dinoréburnaménin 14-ol**

On opère comme à l'exemple 3 à partir de 8,7 g de (±) 10-méthoxy 20,21-dinoréburnaménin-14(15H)-one et obtient 5,73 g de produit brut. On le recristallise dans le tétrahydrofuranne et isole 3,64 g de produit attendu. F = 225¤C.
Spectre RMN :

OH équatorial

3,75 (s) : $OCH_3$

4,15 (m) : >C Ⓗ - N jonction cis

5,47 (dt J = 9 - 5,5 - 5,5) : >C Ⓗ OH axial

6,32 (d, J = 9) : OH équatorial

6,68 (dd J = 2,5 et 9) : $H_6$ ⎫
6,88 (d J = 2,5) : $H_4$ ⎬ indole
7,54 (d J = 9) : $H_7$ ⎭

**Exemple 27 : [(±) (14béta 16alpha)] 14,15-dihydro 10-méthoxy 20,21-dinoréburnaménin 14-ol**

On opère comme à l'exemple 3 à partir de 3,67 g de [(±) (16alpha)] 10-méthoxy 20,21-dinoréburnaménin 14-(15H)-one et obtient 3,7 g de produit brut. On reprend celui-ci par un mélange chloroforme-méthanol (2-1), filtre, puis concentre. On prépare une solution du produit obtenu dans 150 cm3 de tétrahydrofuranne, ajoute lentement 50 cm3 d'éther isopropylique. On essore 2,41 g de produit attendu.
Spectre RMN (DMSO 250 MHz ppm) :

3,74 (s) : $OCH_3$

5,43 (dt, J = 5,5 et 9) : >C Ⓗ OH axial

6,68 (dd, J = 2 et 9) : $H_6$ ⎫
6,87 (d J = 2) : $H_4$ ⎬ indole
7,53 (d J = 9 : $H_7$ ⎭

1,1 à 3,05 : les autres protons

**Exemple 28 : [(±) (14alpha, 16alpha)] 14,15-dihydro 10-méthoxy 20,21-dinoréburnaménin 14-ol**

On met en suspension 4,8 g de [(±)(14béta, 16alpha)] 14,15-dihydro 10-méthoxy 20,21-dinoréburnaménin-14-ol dans 50 cm3 de soude 5N et 50 cm3 de méthanol et porte au reflux pendant 2 heures 30 minutes. A température ambiante, on essore, lave abondamment à l'eau jusqu'à pH neutre des eaux de lavage puis par 100 cm3 de méthanol. On obtient 4,12 g de produit brut que l'on porte au reflux pendant 15 heures dans les mêmes conditions que précédemment. On reprend le produit récupéré par un mélange chloroforme-méthanol (2-1), filtre, concentre, sèche et obtient 3,85 g de produit attendu.
Spectre RMN (DMSO 250 MHz ppm) :

OH axial
3,74 (s) : $OCH_3$
6,11 (d J = 7) : OH

5,83 : ⋝C Ⓗ- OH équatorial

7,31 : (d, J = 8,5) $H_7$ ⎫
6,84 : (d, J = 2,5) $H_4$ ⎬ indole
6,68 : (dd, J = 2,5 et 8,5) $H_6$ ⎭

1,05 à 3,05 les autres protons

**<u>Exemple 29</u> : <u>Hémifumarate de [(±) (16alpha)] 10-méthoxy 20,21-dinoréburnaménine</u>**

On opère comme à l'exemple 2 à partir de 1 g de [(±)(14béta, 16alpha)] 14,15-dihydro 10-méthoxy 20-21-dinoréburnaménin 14-ol. On filtre la solution obtenue, amène à sec sous pression réduite, empâte le résidu avec une solution de bicarbonate de soude, essore, lave abondamment à l'eau jusqu'à pH neutre des eaux de lavage, sèche sous pression réduite à 70¤C et reprend le résidu par 20 cm3 d'éther de pétrole (eb 40-70¤C). On obtient 820 mg de produit sous forme de base F = 145¤C. A partir de 1,7 g de base dissoute dans 50 cm3 d'acétate d'éthyle, on prépare le fumarate. On obtient 1,55 g de produit attendu F = 212¤C.
<u>Spectre RMN $CDCl_3$ 250 MHz (ppm)</u> :

3,85 (s) : $OCH_3$
5,04 (dd J = 2 et 7,5) : H éthylénique en béta de N
6,90 (dd J = 3 et 7,5) : H éthylénique en alpha de N

6,81 (dd J = 2,5 et 8,5) : $H_6$ ⎫
6,94 (d J = 2,5) : $H_4$ ⎬ indole
7,21 (d J = 8,5) : $H_7$ ⎭

1,3 à 3,2 : les autres protons

**<u>Exemple 30</u> : <u>Hémifumarate de la (±) 10-méthoxy 20,21-dinoréburnaménine</u>**

On opère comme à l'exemple 6 à partir de 2,02 g de [(±) (14béta)] 14,15-dihydro 10-méthoxy 20,21-dinoréburnaménin 14-ol et obtient 1,4 g de produit sous forme de base. F = 134¤C. On prépare le fumarate à partir de 1,32 g de ce dernier et isole 1,14 g de produit attendu F = 190¤C.
<u>Spectre RMN ($CDCl_3$ 250 MHz (ppm))</u> :

3,86 (s) : $OCH_3$
4,54 (m) : ⋝C Ⓗ- N jonction cis

6,89 (d, J = 8) : N-C(H)-C

5,27 (dd J = 6 et 8) : -C(H)-CH

6,83 (dd J = 2,5 et 8,5) : $H_6$ ⎫
6,95 (d J = 2,5) : $H_4$ ⎬ indole
7,21 (d J = 8,5) : $H_7$ ⎭

0,90 (m) : 1H ⎫
1,45 à 3,40 ⎭ autres protons

En utilisant les procédés décrits ci-dessus, on a préparé les exemples suivants :

**Exemple 31 : [(±) (14alpha, 16alpha)] 9,11-dichloro 14,15-dihydro 20, 21-dinoréburnaménin-14-ol**

F = 260¤C Rf 0,5 sur alumine (chlorure de méthylène acétone (8-2).

**Exemple 32 : [(±) (14béta, 16alpha)] 10, 11-diméthoxy 14,15-dihydro 20,21-dinoréburnaménin-14-ol**

F = 252¤C

**Exemple 33 : [(±) (14béta, 16alpha)] 9, 10, 11-triméthoxy 14,15-dihydro 20,21-dinoréburnaménin-14-ol**

F = 240¤C

**Exemple 34 : [(±) (14béta, 16alpha)] 9-méthoxy 14,15-dihydro 20,21-dinoréburnaménin-14-ol**

F = 233¤C

En effectuant une déshydratation, comme décrite ci-dessus, sur les alcools obtenus aux exemples 32, 33 et 34, on a préparé les produits suivants sous forme de sels.

**Exemple 35 : Maléate acide de [(±) (16alpha)] 10,11-diméthoxy 20,21-dinoréburnaménine**

F = 216¤C (sel) F = 208¤C (base).

**Exemple 36 : Fumarate neutre de [(±) (16alpha)] 9,10,11-triméthoxy 20,21-dinoréburnaménine**

F = 211¤C (sel) F = 130¤C (base).

**Exemple 37 : Maléate acide de [(±) (16alpha)] 9-méthoxy 20,21-dinoréburnaménine**

F = 205¤C (sel) F = 146¤C (base).

**Exemple 38 : [(±) (16alpha)] 11-nitro 14,15-dihydro 20,21-dinoréburnaménin-14-ol.**

On ajoute par petites fractions 2 g d'hydroborure de sodium dans une solution comprenant 2 g de [(±) (16alpha)] 11-nitro 20,21-dinoréburnaménin14(15H)-one et 100 cm3 de méthanol. On chauffe au reflux pendant 50 minutes, ajoute 200 cm3 d'eau glacée, essore le précipité, le lave à l'eau, le sèche à 75¤C sous pression réduite et obtient 1,75 g de produit attendu. F≧ 260¤C.

Spectre RMN (DMSO 300 MHz ppm) :

5,70 (m) 1/5 H H axial ⎫
6,05 (d) 4/5 H H équatorial ⎭ N-CH-OH

6,65 (d) équatorial ⎫
6,93 (d) axial ⎭ CH-OH

7,53 (d) $H_4$ ⎫
7,93 (dd) $H_5$ ⎬ indole
8,46 (d) $H_7$ ⎭

1,10 à 3,10 : les autres protons.

**Exemple 39 : Maléate acide de [(±) (16alpha)] 11-nitro 20,21-dinoréburnaménine.**

On ajoute 60 mg de trifluorométhane sulfonate de cuivre dans une suspension comprenant 1,25 g de produit obtenu à l'exemple 38 dans 125 cm3 de xylène. On chauffe 15 heures au reflux, filtre et élimine le solvant sous pression réduite. On chromatographie le résidu sur silice (éluant : acétate d'éthyle) et obtient 990 mg de produit sous forme de base. F = 172¤C. On dissout 1,24 g de base préparée comme ci-dessus dans 200 cm3 d'un mélange éthanol-acétate d'éthyle (1-1), ajoute 487 mg d'acide maléique dissous à chaud dans 50 cm3 d'éthanol, agite pendant 2 heures à température ambiante, essore le précipité, le lave à l'éthanol et le sèche à 70¤C sous pression réduite. On récupère 1,44 g de produit attendu. F > 260¤C.
Spectre RMN ($CDCl_3$ 250 MHz ppm) :

5,27 (dd, J = 2 et 8)
7,00 (dd, J = 3 et 8) 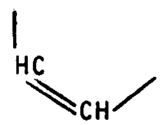 Delta Z

7,47 (d, J = 8,5) $H_4$ ⎫
8,00 (dd, J = 2 et 8,5) $H_5$ ⎬ indole
8,26 (d, J = 2) $H_7$ ⎭

1,35 à 3,25 : les CH et $CH_2$.

**Exemple 40 : [(±) (16alpha)] 9-nitro 14,15-dihydro 20,21-dinoréburnaménin-14-ol.**

On opère comme à l'exemple 38 en utilisant 2 g de [(±) (16alpha)] 9-nitro 20,21 dinoréburnaménin-14-(15H)-one et obtient 1,9 g de produit attendu. F > 260¤C.
Spectre RMN (DMSO 250 MHz ppm) :

| | | | | |
|---|---|---|---|---|
| 5,71 | (m) | H axial | ) | 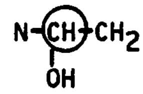 |
| 6,03 | (d) | H équatorial | ) | |
| 6,49 | (d) | OH axial | | |
| 6,75 | (d) | OH équatorial | | |
| 7,24 | (t) | $H_6$ | ) | |
| 7,89 | (d) | $H_7$ | ) indole | |
| 8,10 | (d) | $H_5$ | ) | |

1,15 à 3,2 : les CH et $CH_2$.

**Exemple 41 : Maléate acide de [(±) 16alpha)] 9-nitro 20,21-dinoréburnaménine.**

On opère comme à l'exemple 39 à partir de 1,9 g de produit obtenu à l'exemple 40, 100 mg de trifluorométhane sulfonate de cuivre et 200 cm3 de xylène. On obtient 1,65 g de produit sous forme de base F = 198¤C. On utilise 1,57 g de base et 615 mg d'acide maléïque et obtient 1,42 g de maléate acide attendu. F = 228¤C.
Spectre RMN ($CDCl_3$ 250 MHz ppm) :

de 1,36 à 3,41 (m) : 12 H

| | | | |
|---|---|---|---|
| 5,28 | (dd, J = 12 et 8) | ) CH=CH | |
| 6,99 | (dd, J = 3 et 8) | ) | |
| 7,19 | (t, J = 8) | $H_6$ | ) |
| 7,59 | (dd, J = 8 et 1) | $H_7$ | ) indole |
| 7,97 | (dd, J = 8 et 1) | $H_5$ | ) |

**Exemple 42 : [(16alpha) (±)] 14,15-dihydro 11-diméthylamino 20,21-dinoréburnaménin-14-ol.**

On ajoute par petites fractions 1 g d'hydroborure de sodium dans une suspension comprenant 1,94 g de [(16alpha) (±)] 11-diméthylamino 20,21-dinoréburnaménin 14(15H)-one préparé comme à la préparation 4 et 200 cm3 de méthanol. On chauffe au reflux pendant 2 heures, refroidit à température ambiante, ajoute de nouveau 1 g d'hydroborure de sodium et agite 2 heures au reflux. On ajoute 500 cm3 d'eau glacée, essore le précipité, le lave à l'eau, le sèche à 80¤C sous pression réduite et obtient 1,64 g de produit attendu. F ≃ 260¤C.
Spectre RMN (DMSO 300 MHz ppm) :

| | | |
|---|---|---|
| 5,41 | (m, dd, J = 6 et 9) | OH axial |
| 5,84 | (d, s) | OH équatorial |

| | | | |
|---|---|---|---|
| 6,37 | (d) | | OH |
| 6,09 | (d) | | |

| | | | |
|---|---|---|---|
| 6,62 | (td) | $H_5$ | indole |
| 6,79 | (dd) | $H_7$ | |
| 7,03 | (d) | $H_7$ | |
| 7,17 | (d) | $H_4$ | |

2,87 (s) : les $CH_3$

de 1,1 à 3,1 : les autres protons.

**Exemple 43 : Maléate de [(16alpha) (±)] N,N-diméthyl 20,21-dinoréburnaménin-11-amine.**

On ajoute 20 mg d'acide para-toluènesulfonique dans une suspension comprenant 400 mg de produit préparé à l'exemple 42 dans 40 cm3 de toluène. On chauffe 15 heures au reflux, concentre à sec, chromatographie le résidu sur silice (éluant : acétate d'éthyle) et obtient 290 mg de produit sous forme de base. F = 132¤C. On dissout 900 mg de base préparée comme ci-dessus dans 50 cm3 d'acétate d'éthyle et 10 cm3 d'éthanol, ajoute 712 mg d'acide maléïque en solution dans 10 cm3 d'éthanol bouillant. On maintient 3 heures sous agitation à température ambiante, essore le précipité, le lave à l'éthanol et le sèche sous pression réduite à 70¤C. Onobtient 1 g de maléate attendu. F = 228¤C.
Spectre RMN ($CDCl_3$ 250 MHz ppm) :

2,95 (s) 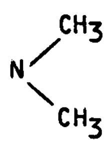

5,01 (d,d, J = 2 et 7,5) : N-CH=C(H)
6,94 (d,d, J = 2 et 7,5) : N-C(H)=CH

| | | | |
|---|---|---|---|
| 6,68 | (s) | $H_7$ | indole |
| 6,70 | (d,d J = 7,5 et 2) | $H_5$ | |
| 7,28 | (d, J = 7,5) | $H_4$ | |

**Exemple 44 : [16alpha) (±)] N-(14,15-dihydro 14-hydroxy 20,21-dinoréburnaménin-11-yl) acétamide.**

On ajoute par petites fractions 1,75 g d'hydroborure de sodium dans une suspension comprenant 1,75 g de [(16alpha) (±)] N-(14,15-dihydro 14-oxo 20,21-dinoréburnaménin-11-yl) acétamide préparé comme indiqué à la préparation 5 dans 100 cm3 de méthanol. On chauffe 3 heures au reflux, refroidit à

température ambiante, ajoute 200 cm3 d'eau glacée, essore le précipité, le lave à l'eau, l'empâte dans 50 cm3 de méthanol, le sèche à 80¤C sous pression réduite et obtient 1,3 g de produit attendu. F > 260¤C.
Spectre RMN (DMSO 300 MHz ppm) :

2,03 : Ac
5,99 (t) : H axial N-CH-OH
5,75 : H équatorial
6,00 : H mobile

7,09 à 7,25 $H_4$ et $H_5$ ) indole
7,80 $H_7$

9,81 : H mobile
1,15 à 2,98 : les autres $CH_2$ et CH.

**Exemple 45 : Maléate acide de [(16alpha) (±)] N-20,21-dinoréburnaménin-11-yl) acétamide.**

On ajoute 60 mg d'acide para-toluènesulfonique à une suspension comprenant 1,2 g de produit préparé à l'exemple 44 dans 150 cm3 de toluène et chauffe 16 heures au reflux ; on refroidit, filtre, élimine le solvant sous pression réduite, chromatographie le résidu sur silice (éluant : chlorure de méthylène-méthanol (95-5) et obtient 1 g de produit sous forme de base. F = 208¤C. On met en solution 1,2 g de produit obtenu comme ci-dessus dans 100 cm3 d'acétate d'éthyle et ajoute 453 mg d'acide maléïque préalablement dissous dans 50 cm3 d'acétate d'éthyle et 10 cm3 d'éthanol. On agite 4 heures à température ambiante, essore, sèche & 500¤C sous pression réduite et obtient 1,22 g de maléate acide attendu. F = 217¤C.
Spectre RMN ($CDCl_3$ 300 MHz ppm) :

2,19 (s) : 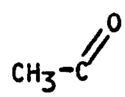
6,97 (dd, J = 7,5 et 3) : N- C(H)=CH
5,08 (dd, J = 7,5 et 2) : N-CH=C(H)
7,24 : NH

7,96 (d, J = 2) $H_7$ ) indole
7,34 (d, J = 8) $H_4$
6,88 (dd, J = 2 et 8) $H_5$

de 1,4 à 3,2 : les autres protons.

**Exemple 46 : Maléate acide de [(16alpha) (±)] 20,21-dinoréburnaménin-11-amine.**

On ajoute 3 cm3 d'une solution aqueuse à 50% d'hydroxyde de potassium dans 1,5 g de la base obtenue à l'exemple 45 en solution dans 100 cm3 d'éthanol. On chauffe 24 heures au reflux, ajoute 200 cm3 d'eau, essore le précipité et le reprend dans l'acétate d'éthyle. On lave à l'eau jusqu'à neutralité cette phase oryanique, la sèche, élimine le solvant, chromatographie le résidu sur silice (éluant : acétate d'éthyle-triéthylamine 9-1) et obtient après sèchage à 65¤C, 1 g de produit sous forme de base. F = 252¤C. On dissout 850 mg de cette base dans un mélange de 100 cm3 d'acétate d'éthyle et 20 cm3 d'éthanol, ajoute 743 mg d'acide maléïque préalablement dissous dans 10 cm3 d'éthanol, agite pendant 3 heures à température ambiante, essore le précipité, le lave à l'éthanol, le sèche à 50¤C sous pression réduite. On récupère 1 g de produit attendu, F = 250¤C, que l'on purifie par empâtage dans un mélange acétate d'éthyle-éthanol (1-1).

Spectre RMN ($CDCl_3$ 300 MHz ppm) :

5,01 (dd, J = 7,5 et 2) : N-CH=CH
6,84 (dd, J = 7,5 et 2) : N-CH=CH

6,52 (dd, J = 8,5 et 2) $H_5$
6,64 (d, J = 2) $H_7$ indole
7,22 (d, J = 8,5) $H_4$

3,50 (ep) : 2H mobiles
de 1,30 à 3,18 : les autres protons.

**Exemple 47 : [(16alpha) (±)] 11-bromo 14,15-dihydro 20,21-dinoréburnaménin-14-ol.**

On ajoute par petites fractions 412 mg d'hydroborure de sodium dans une suspension comprenant 750 mg de [(±) (16alpha)] 11-bromo 20,21-dinoréburnaménin-14(15H)-one préparé comme indiqué dans le brevet belge n¤ 44087 B) et 20 cm3 de méthanol. On ajoute 184 mg de chlorure de lithium, agite pendant 2 heures à température ambiante puis 30 minutes à 40¤C. On verse le milieu réactionnel sur 100 cm3 d'eau glacée, essore le précipité, le lave à l'eau, le sèche sous pression réduite à 80¤C et obtient 711 mg de produit attendu. F = 245¤C.

Spectre RMN (DMSO 400 MHz ppm) :

5,52 (m, dd après échange) : axial CH-OH
5,88 (d, J = 6,5) : équatorial CH-OH

6,61 (d, J = 8,5) : OH

7,13 (dd, dédoublé) $H_5$
7,31 (d, dédoublé) $H_4$ indole
7,83 (d, J = 1,5) $H_7$

**Exemple 48 : Maléate acide de [(±) (16alpha)] 11-bromo 20,21-dlnoréburnaménine.**

On ajoute 80 mg d'acide para-toluènesulfonique à une suspension comprenant 1,5 g de produit préparé comme à l'exemple 47 dans 100 cm3 de toluène et chauffe 15 heures au reflux. On filtre, amène à sec, chromatographie le résidu sur silice (éluant : acétate d'éthyle) et obtient après séchage à 50¤C sous pression réduite 1,14 g de produit sous forme de base. F = 133¤C. On dissout 1,08 g de base dans 100 cm3 d'acétate d'éthyle et 20 cm3 d'éthanol, agite 3 heures à température ambiante, essore le précipité, le lave à l'acétate d'éthyle, le sèche à 70¤C sous pression réduite et récupère 1,33 g de maléate acide attendu. F = 247¤C.

Spectre RMN ($CDCl_3$ 400 MHz ppm) :

5,12 (dd, J = 7,5 et 2) : CH

6,88 (dd, J = 7,5 et 3) : C H -N

| | | |
|---|---|---|
| 7,18 (dd, J = 8,5 et 2) | $H_5$ | indole |
| 7,30 (dd, J = 8,5) | $H_4$ | |
| 7,45 (d, J = 2) | $H_7$ | |

autres protons :

1,42 (m) : 1H ; 1,87 (m) : 2H ; 1,98 (m) : 1H ; 2,29 à 2,41 (m) : 2H ; 2,61 (m) : 1H ; 2,70 (m) : 1H ; 2,89 (dl): 1H ; 2,97 à 3,08 (m) : 2H ; 3,14 (dd, J = 11 et 5,5) : 1H.

**Exemple 49 : [(±) (16alpha)] 14,15-dihydro 11-éthyl 20,21-dinoréburnaménin-14-ol.**

On ajoute par petites fractions 840 mg d'hydroborure de sodium puis 370 mg de chlorure de lithium dans une suspension comprenant 1,3 g de [(16alpha) (±)] 11-éthyl 20,21-dinoréburnaménin-14(15H)-one préparé comme indiqué à la préparation 6 et 100 cm3 de méthanol. On chauffe 6 heures au reflux, ajoute 300 cm3 d'eau glacée, essore le précipité formé, le lave à l'eau, puis le sèche à 100¤C sous pression réduite. On purifie le produit brut par empâtage dans le méthanol et recueille 980 mg de produit attendu. F = 247¤C.

Spectre RMN (DMSO 300 MHz ppm) :

5,47 (m) axial : N-CH-OH

5,88 équatorial N-CH-OH

6,40 (d) : OH

7,50 (d) $H_7$

7,25 (m) $H_4$ et $H_7$ ) indole

6,88 (m) $H_5$

1,22 (m) : $CH_3$-$CH_2$

1,0 à 3,1 (m) : les autres protons.

**Example 50 : Maléate acide de [(±) (16alpha)] 11-éthyl 20,21-dinoréburnaménine.**

On ajoute 50 mg d'acide para-toluènesulfonique à 940 mg de produit obtenu à l'exemple 49 en suspension dans 100 cm3 de toluène puis chauffe 1 heure au reflux. On concentre à sec la solution, chromatographie le résidu sur silice (éluant : chlorure de méthylène-méthanol 97-3). On obtient 870 mg de produit attendu sous forme de base. F = 131¤C. On dissout 770 mg de cette base dans 50 cm3 d'acétate d'éthyle et 20 cm3 d'éthanol, ajoute 320 mg d'acide maléïque préalablement dissous dans 10 cm3 d'éthanol. On agite 2 heures à température ambiante, essore et sèche sous pression réduite à 60¤C. On récupère 910 mg du maléate attendu. F = 176¤C.
Spectre RMN ($CDCl_3$ 300 MHz ppm) :

1,28 (t) : $CH_3$-$CH_2$

2,75 (q) : $CH_3$-$CH_2$

5,05 (dd, J = 7,5 et 2): N-CH=CH

6,95 (m) : $H_5$ et autre éthylénique

7,15 (s) : $H_7$

7,36 (d, J = 8) : $H_4$

1,41 (m)
1,80 à 3,18 ) les autres protons

**Exemple 51 : [(16alpha) (±)] 14,15-dihydro 11-éthoxy 20,21 dinoréburnaménir-14-ol.**

On ajoute par petites fractions 160 mg de borohydrure de lithium dans une suspension comprenant 464 mg de [(16alpha) (±)] 11-éthoxy 20,21-dinoréburnaménin-14(15H)-one préparé comme indiqué à la préparation 7 et 20 cm3 de méthanol. On agite 1 heure à température ambiante, ajoute 100 cm3 d'eau, essore le précipité, le lave à l'eau, le sèche à 70¤C sous pression réduite et obtient 385 mg de produit attendu. F = 218¤C.
Spectre RMN (DMSO 400 MHz ppm) :

1,34 (t) ⎫
4,00 (q) ⎭ OEt

5,43 (m, dd après échange) : axial C(H)-OH
5,83 (m, dd après échange) : équatorial C(H)-OH
6,44 (d, J = 8,5) : CH-O(H)
7,21 (m) : $H_4$ et $H_7$ de l'indole
de 1,10 à 3,00 (m) : les autres protons.

**Exemple 52 : Maléate acide de [(16alpha) (±)] 11-éthoxy 20,21-dinoréburnaménine.**

On ajoute 20 mg d'acide para-toluènesulfonique dans une suspension comprenant 350 mg de produit obtenu à l'exemple 51 et 20 cm3 de toluène. On chauffe 15 heures au reflux, filtre, concentre à sec sous pression réduite et chromatographie le résidu sur silice (éluant : acétate d'éthyle). On obtient 286 mg de produit sous forme de base. F = 105¤C. On dissout 260 mg de base dans 50 cm3 d'acétate d'éthyle, ajoute 103 mg d'acide maléïque dissous dans 10 cm3 d'acétate d'éthyle, agite 3 heuresà température ambiante, essore et sèche à 70¤C sous pression réduite. On récupère 315 mg de produit attendu. F = 211¤C.
Spectre RMN ($CDCl_3$ 400 MHz ppm) :

1,43 (t) ⎫
4,07 (q) ⎭ OEt

5,05 (dd, J = 7,5 et 2) ⎫
6,89 (dd, J = 7,5 et 3) ⎭ 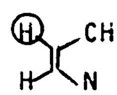

6,74 (dd, J = 8 et 2) $H_5$ ⎫
6,84 (d, J = 2) $H_7$ ⎭ indole

1,3 à 3,15 : les autres protons.

**Exemple 53 : [(16alpha) (±)] 14,15-dihydro 11-hydroxy 20,21-dinoréburnaménin-14-ol.**

On opère comme à l'exemple 51 en utilisant 900 mg de [(16alpha) (±)] 11-hydroxy 20,21-dinoréburnamin-14(15H)-one dans 90 cm3 de toluène et 5,3 cm3 d'hydrure de diisobutylaluminium à la place de l'hydroborure de sodium. On obtient 360 mg de produit attendu (mélange d'épimères). F = 240¤C.
Spectre RMN (DMSO 300 MHz ppm) :

5,34 (dt, J = 6 et 9) : C(H)-OH axial (20%)
5,74 (d) : C(H)-OH équatorial (80%)

6,02 (d, J = 7) ) CH-O(H)
6,26 (d, J = 9) )

6,52 (dd) $H_5$ )
6,79 (d) $H_7$ )
7,07 (d) $H_7$ ) indole
7,10 (d) $H_4$ )
7,11 (d) $H_4$ )

8,83 (s) mobile : OH phénol
1,05 à 3,05 (m) : les autres protons.

**Exemple 54 : [(16alpha) (±)] 11-hydroxy 20,21-dinoréburnaménine.**

On opère comme à l'exemple 52 au départ de 360 mg de produit obtenu à l'exemple 53 en utilisant quelques mg de trifluorométhane sulfonate de cuivre dans 20 cm3 de toluène. On obtient 250 mg de produit attendu. F > 260¤C.
Spectre RMN (DMSO 300 MHz ppm) :

5,05 (dl, J = 7) : N-CH=C(H)
6,56 (dd, J = 7 et 2) : N-C(H)=CH

6,88 (sl) $H_7$ ) indole
7,17 (m) $H_5$ et $H_4$ )

9,06 (s) : OH
de 1,2 à 3,1 (m) : les autres protons.

**Exemple 55 : Maléate de (3alpha) 11-méthyl 20,21-dinoréburnaménine.**

**Stade A :** (3alpha) (-) 11-méthyl 20,21-dinoréburnaménin-14(15H)-one.

On dissout à chaud 3,6 g de [(±) (16alpha)] 11-méthyl 20,21-dinoréburnaménin-14(15H)-one (trans,dl) dans 50 cm3 d'éthanol. On ajoute 2,04 g d'acide (-) di-O,O'-pivaloyl L-tartrique en solution dans 22 cm3 d'éthanol, agite 2 minutes, maintient 16 heures à température ambiante, essore le produit cristallisé, le lave à l'éthanol, le sèche à 60¤C sous pression réduite et obtient 4,67 g de produit brut que l'on recristallise dans le méthanol. On récupère 2,17 g de sel intermédiaire [F = 260¤C ; [$alpha_D$] = -137,5¤ ± 3,5¤ (c = 0,5% diméthylformamide)] que l'on met en suspension dans 50 cm3 d'eau et 50 cm3 d'acétate d'éthyle. On ajoute 5 cm3 d'ammoniaque et agite 30 minutes. On sépare les phases, lave la phase organique à l'eau, la sèche et élimine le solvant sous pression réduite. On obtient 1,38 g de l'énantiomère 3alpha attendu. F = 198¤C.
[$alpha_D$] = -162,5¤ ± 3,5¤ ( c = 0,5% chloroforme)

**Stade B :** (3alpha) 11-méthyl 20,21-dinoréburnaménine.

On ajoute en 5 minutes 1,24 g d'hydroborure de sodium dans 1,53 g de produit préparé comme indiqué au stade A en suspension dans 30 cm3 de méthanol à 10% d'eau. On chauffe 7 heures au reflux, ajoute 30 cm3 d'eau et 1 cm3 d'acide acétique. On agite 15 minutes à température ambiante, ajoute 2 cm3 d'ammoniaque puis agite de nouveau 15 minutes. On essore le précipité, le lave à l'eau jusqu'à neutralité et le sèche sous pression réduite à 60¤C. On obtient 1,41 g de produit (mélange d'OH axial et équatorial) que l'on met en suspension dans 28 cm3 de toluène. On ajoute 70 mg d'acide para-toluène sulfonique et chauffe 16 heures à 100¤C. On obtient une solution que l'on concentre sous pression réduite. On chromatographie le résidu sur silice (éluant : chlorure de méthylène-acétone 9-1) et obtient 1,20 g de produit attendu. F = 134¤C.
[$alpha_D$] = -433 ± 6¤ (c = 0,5% chloroforme)

**Stade C :** Maléate de (3alpha) 11-méthyl 20,21-dinoréburnaménine.

On dissout 1,6 g de produit préparé comme au stade B dans 60 cm3 d'acétate d'éthyle, ajoute 0,702 g d'acide maléïque en solution dans 15 cm3 d'acétate d'éthyle et agite 1 heure à température ambiante. On essore le précipité, le sèche sous pression réduite à température ambiante. On obtient 2,120 g de maléate attendu. F = 195¤C.

| Analyse : $C_{18}H_{20}N_2$, $C_4H_4O_4$ : 380,448 | | | | | | |
|---|---|---|---|---|---|---|
| Calculé<br>Trouvé | C% | 69,46<br>69,6 | H% | 6,36<br>6,5 | N% | 7,36<br>7,3 |

**Exemple 56 : Maléate de (16alpha) 11-méthyl 20,21-dinoréburnaménine.**

**Stade A :** [(16alpha) (±)] 11-méthyl 20,21-dinoréburnaménin-14(15H)-one.

On reprend les eaux mères éthanoliques et méthanoliques obtenues à l'exemple 55, Stade A, lors de la cristallisation de l'énantiomère 3alpha et les concentre à sec. On reprend le résidu par 250 cm3 d'acétate d'éthyle et 150 cm3 d'eau, ajoute 10 cm3 d'ammoniaque et agite 30 minutes. On sépare les phases, lave à l'eau la phase organique et concentre sous pression réduite. On récupère 2,3 g de [(±) (16alpha)] 11-méthyl 20,21-dinoréburnamé-nin-14(15H)-one enrichi en énantiomère (16alpha). On poursuit la synthèse en opérant comme au stade A de l'exemple 55 en utilisant les 2,3 g du produit préparé ci-dessus, 1,3 g d'acide (+) di-O,O'-pivaloyl-D-tartrique. On obtient 1,93 g de sel intermédiaire [F = 260¤C, [$alpha_D$] = +109,5¤ ± 3¤ (c = 0,4% diméthylformamide)] puis 1,22 g du produit attendu (énantiomère 16 alpha). F = 198¤C.
[$alpha_D$] = +163,5¤ ± 3,5¤ (c = 0,5% chloroforme).

**Stade B :** (16alpha) 11-méthyl 20,21-dinoréburnaménine.

On opère comme au stade B de l'exemple 55 à partir de 1,53 g de produit préparé comme au stade A ci-dessus. On obtient 1,17 g de produit réduit (mélange d'OH axial et équatorial) puis 1,05 g de produit déshydraté attendu. F = 134¤C.
[$alpha_D$] = +435,5¤ ± 6¤ (c = 0,5% chloroforme).

**Stade C :** Maléate de (16alpha) 11-méthyl 20,21-dinoréburnaménine.

On opère comme au stade C de l'exemple 55 à partir de 1,52 g du produit obtenu au stade B ci-dessus. On obtient 2 g de maléate attendu. F = 195¤C.

| Analyse : $C_{18}H_{20}N_2$, $C_4H_4O_4$ : 380,448 | | | | | | |
|---|---|---|---|---|---|---|
| Calculé<br>Trouvé | C% | 69,46<br>69,3 | H% | 6,36<br>6,4 | N% | 7,36<br>7,3 |

**Exemple 57 : (3alpha) 11-méthyl 20,21-dinoréburnaménine.**

On opère comme au stade A de l'exemple 55 en utilisant au départ 3,2 g de [(±) (16alpha)] 11-méthyl 20,21-dinoréburnaménine obtenu à l'exemple 10 dans 32 cm3 d'acétate d'éthyle et 1,93 g d'acide (-) di-O,O'-pivaloyl-L-tartrique en solution dans 19 cm3 d'acétone. On obtient 1,15 g de sel intermédiaire [F = 215¤C, [$alpha_D$] = -340,5¤ ± 5,5¤ (c = 0,5% diméthylformamide)] puis 0,7 g de l'énantiomère 3alpha attendu. F = 134¤C.
[$alpha_D$] = -466,5¤ ± 6¤ (c = 0,5% chloroforme).

**Exemple 58 : (16alpha) 11-méthyl 20,21-dinoréburnaménine.**

On opère comme au stade A de l'exemple 56 à partir des eaux-mères obtenues lors de la synthèse de l'énantiomère 3alpha à l'exemple 57. On obtient 2,45 g de [(±) (16alpha)] 11-méthyl 20,21-dinoréburnaménin-14(15H)-one enrichi en énantiomère 16alpha puis poursuit la synthèse en utilisant 1,48 g d'acide (+) di-O,O'-pivaloyl-D-tartrique. On obtient 1,33 g de sel intermédiaire [F = 215¤C, [$alpha_D$] = +305,5¤ ± 5¤ (c = 0,5% diméthylformamide)] puis 0,810 g de l'énantiomère 16alpha attendu. F = 134¤C.
[$alpha_D$] = +447,5¤ ± 6¤ (c = 0,5% chloroforme).

**Exemple 59 : (3alpha) 11-chloro 20,21-dinoréburnaménine.**

On opère comme au stade A de l'exemple 55 en utilisant au départ 4,6 g de (±) 11-chloro 20,21-dinoréburnaménine préparé comme à l'exemple 2 et 2,57 g d'acide (-) di-O,O'-pivaloyl-L-tartrique en solution dans 50 cm3 d'acétate d'éthyle. On obtient 1,70 g de sel intermédiaire [F = 215¤C, [$alpha_D$] -321¤ ± 5¤ (c = 0,5% diméthylformamide)] puis 1,06 g de l'énantiomère 3alpha attendu. F = 145¤C.
[$alpha_D$] = -441¤ ± 5¤ (c = 1% chloroforme)

En utilisant l'acide oxalique, on a préparé l'oxalate. F ≃ 135¤C.

| Analyse : | | | | | | | | |
|---|---|---|---|---|---|---|---|---|
| Calculé<br>Trouvé | C% | 60,88<br>60,7 | H% | 5,11<br>5,2 | N% | 7,47<br>7,2 | Cl% | 9,46<br>9,4 |

**Exemple 60 : (16alpha) 11-chloro 20,21-dinoréburnaménine.**

On opère comme au stade A de l'exemple 56 à partir des eaux mères obtenues lors de la synthèse de l'énantiomère 3alpha à l'exemple 59. On obtient 3,5 g de (±) 11-chloro 20,21-dinoréburnaménine enrichi en énantiomère 16 alpha, puis poursuit la synthèse en utilisant 1,95 g d'acide (+) di-O,O'-pivaloyl-D-tartrique. On obtient 1,82 g de sel intermédiaire ( F ≃ 215¤C, [$alpha_D$] = +323¤ ± 6¤ (c = 0,5% diméthylformamide)] puis 1,15 g d'énantiomère 16alpha attendu. F = 145¤C.
[$alpha_D$] = +449¤ ± 5¤ (c = 1% $CHCl_3$).

En utilisant l'acide oxalique, on a préparé l'oxalate. F # 195¤C.

| Analyse : | | | | | | | | |
|---|---|---|---|---|---|---|---|---|
| Calculé<br>Trouvé | C% | 60,88<br>60,7 | H% | 5,11<br>5,1 | N% | 7,47<br>7,4 | Cl% | 9,46<br>9,5 |

**Préparation 1 : [(±) 16alpha] 11-chloro 20,21-dinoréburnaménin-14(15H)-one utilisée au départ de l'exemple 1.**

**Stade A :** 1-[2-(6-chloro) 1H-indol-3-yl) éthyl] 2-pipéridinone.

On porte au reflux 22,37 g de 6-chloro tryptamine, 9,21 g de carbonate de potassium dans 200 cm3 d'éthoxy éthanol et introduit en 3 heures 30 minutes 21 cm3 de bromovalérate d'éthyle dans 42 cm3 d'éthoxy éthanol et maintient le reflux encore 1 heure. Après refroidissement, on essore le précipité, lave

par du méthanol et concentre à sec le filtrat. On empâte le résidu dans 50 cm3 d'acide chlorhydrique 2N, puis dans 150 cm3 d'eau. On lave abondamment à l'eau le produit solide obtenu jusqu'à pH neutre des eaux de lavage. On le reprend par du chlorure de méthylène, sèche la solution organique, amène à sec et empâte le résidu dans 200 cm3 d'éther isopropylique. On obtient 25,5 g de produit attendu F = 191¤C.

**Stade B :** 2,3,4,6,7,12-hexahydro 10-chloro indolo[2,3-a]quinolizine.

On porte à 60¤C 2 g de produit obtenu ci-dessus dans 10 cm3 de dioxanne et ajoute lentement 2 cm3 d'oxychlorure dephosphore, puis maintient l'agitation dans ces conditions pendant encore 1 heure. On verse la suspension, lentement dans une solution à 0¤C constituée par 4 cm3 d'acide perchlorique concentré dans 50 cm3 d'eau et maintient sous agitation à 0¤C encore 1 heure. On dissout le perchlorate obtenu encore humide dans 10 cm3 d'acétone à 40¤C sous atmosphère inerte et à l'abri de la lumière. On introduit en 10 minutes à 40¤C 20 cm3 d'ammoniaque concentré, puis agite pendant 15 minutes à 40¤C. On ajoute 10 cm3 d'eau, refroidit à 0¤C pendant 30 minutes, essore le précipité, le lave abondamment à l'eau, sèche sous pression réduite à 50¤C et obtient 1,54 g de produit attendu F = 165¤C.

**Stade C :**[(±) 16alpha] 11-chloro 20,21-dinoréburnaménin-14(15H)-one utilisée au départ de l'exemple 1.

On dissout 1,38 g de 2,3,4,6,7,12-hexahydro 10-chloro indolo [2,3-a] quinolizine dans 5 cm3 de diméthylformamide, ajoute 0,8 cm3 d'iodoacétate d'éthyle et agite 6 heures à température ambiante. On ajoute avec précaution 5 cm3 d'acide iodhydrique dans 5 cm3 d'eau et porte au reflux pendant 10 heures. Après refroidissement à température ambiante, on ajoute, à la suspension obtenue, 20 cm3 d'eau et de glace et agite 1 heure à 0¤C. On essore, lave à l'eau, met le produit dans 25 cm3 d'un mélange tétrahydrofuranne-méthanol (6-4), refroidit à 0¤C et ajoute par petites fractions du borhydrure de sodium en agitant à 0¤C encore 1 heure. On ajoute lentement 1,5 cm3 d'acide acétique, agite 10 minutes, alcalinise par addition de 3 cm3 d'ammoniaque concentré, ajoute 50 cm3 d'eau et extrait au chlorure de méthylène. On lave la phase organique à l'eau, sèche, concentre à sec et chromatographie le résidu sur silice (éluant chlorure de méthylène-méthanol 95-5). On isole 500 mg de produit attendu F = 205¤C.

En utilisant le même procédé, au départ de tryptamines substituées correspondantes, on a préparé les produits suivants :

- [(±) (16alpha)] 11-méthyl 20, 21-dinoréburnaménin-14(15H)-one utilisée au départ de l'exemple 7.
- [(±) (16alpha)] 10-méthyl 20, 21-dinoréburnaménin-14(15H)-one utilisée à l'exemple 13.
- [(±) (16alpha)] 11-méthoxy 20, 21-dinoréburnaménin-14(15H)-one utilisée au départ de l'exemple 21.
- [(±) (16alpha)] 10-méthoxy 20, 21-dinoréburnaménin-14(15H)-one utilisée au départ de l'exemple 27.
- [(±) (16alpha)] 10-chloro 20, 21-dinoréburnaménin-14(15H)-one utilisée au départ de l'exemple 4.

**Préparation 2 : (±) 10-chloro 20,21-dinoréburnaménin-14(15H)-one utilisée au départ de l'exemple 3.**

**Stade A :** 1-[2-(5-chloro 1H-indol 3-yl) éthyl] 2-pipéridinone.

On opère comme au stade A de la préparation 1 à partir de 57,6 g de 5-chloro tryptamine et obtient 31,5 g de produit attendu F = 152¤C.

**Stade B :** 9-chloro 2,3,4,6,7,12-hexahydro indolo[2,3 a] quinolizine.

On dissout 26,7g de produit obtenu précédemment dans 30 cm3 de diméthylaniline et 150 cm3 de dioxane et opère comme au stade B de la préparation 1. On obtient 22,2 g de produit attendu F = 150¤C utilisé rapidement pour l'étape suivante.

**Stade C :** 9-chloro 1,2,3,4,6,7,12,12b-octahydro indolo[2,3-a] quinolizine 2-acétate d'éthyle

Sous atmosphère inerte et à l'abri de la lumière, on chauffe à 60¤C 100 cm3 de diméthylformamide, ajoute 15,64 g d'iodure de sodium, laisse revenir lentement à 55¤C 11,6 cm3 de bromo acétate d'éthyle et maintient sous agitation à 60¤C pendant 1 heure 30 minutes et laisse revenir lentement à température ambiante. On ajoute l'hydroquinone, puis 18 g du produit obtenu en béta et laisse sous agitation pendant 72 heures. On verse sur 55 cm3 d'acide perchlorique dans un litre d'eau glacée, agite 1 heure à 0¤C, essore, lave à l'eau glacé, sèche sous pression réduite et dissout le perchlorate obtenu dans 300 cm3 d'un

mélange tétrahydrofuranne méthanol (1-1)à moins de 20¤C, ajoute par petites fractions 4 g d'hydroborure de sodium, puis agite 2 heures. On ajoute 20 cm3 d'acide acétique, agite 1/4 d'heure, introduit 40 cm3 d'ammoniaque, puis 500 cm3 d'eau. On extrait cette suspension par de l'acétate d'éthyle, sèche la phase organique, concentre à sec et chromatographie le résidu sur silice (éluant acétate d'éthyle chlorure de méthylène (1-1). On recueille 9,95 g de produit attendu F = 165¤C.

**Stade D :** de la (±) 10-chloro 20,21-dinoréburnaménin-14(15H)-one utilisée au départ de l'exemple 3.

Dans une solution de 7,65 g de produit obtenu en C dans 50 cm3 de méthanol, on ajoute par petites fractions 1,8 g de méthylate de sodium et porte au reflux 45 minutes. A température ambiante, on verse la suspension obtenue sur 50 cm3 d'eau. On essore le précipité, lave à l'eau jusqu'à pH7 des eaux de lavage, sèche sous pression réduite à 100¤C et obtient 5,87 g de produit attendu F = 214¤C.

En procédant d'une manière analogue au départ de tryptamines substituées correspondantes, les produits suivants ont été préparés :

- (±) 11-méthyl 20,21-dinoreburnaménin-14(15H)-one utilisée à l'exemple 9.
- (±) 10-méthyl 20,21-dinoréburnaménin-14(15H)-one utilisée à l'exemple 14.
- (±) 11-méthyl 20,21-dinoréburnaménin-14(15H)-one utilisée à l'exemple 20.
- (±) 10-méthoxy 20,21-dinoréburnaménin-14(15H)-one utilisée à l'exemple 26.

**Préparation 3 : [(±) (16alpha)] 11-nitro 20,21-dinoréburnaménin-14(15H)-one et [(±) (16alpha)] 9-nitro 20,21-dinoréburnaménin-14(15H)-one utilisés au départ des exemples 38 et 40.**

On ajoute une solution de 20 g de (3béta,16alpha) (±) 20,21-dinoréburnaménin-14(15H)-one dans 100 cm3 d'acide acétique à un mélange comprenant 30 cm3 d'acide nitrique et 30 cm3 d'acide acétique en maintenant la température entre 30¤C et 35¤C et l'agitation pendant 1 heure. On prépare ainsi 3 unités opératoires, les rassemble et les verse dans 2 litres d'eau glacée. On alcalinise par addition d'ammoniaque concentré, essore le précipité formé, le lave à l'eau, le sèche à 60¤C sous pression réduite et le chromatographie sur silice (éluant : acétate d'éthyle). On obtient 41,8 g d'isomère 11-nitro (F = 196¤C) et 15,7 g d'isomère 9-nitro (F = 174¤C).

. Isomère 11-nitro.
Spectre RMN ($CDCl_3$ 250 MHz ppm) :

1,29 (m) : 1H
de 1,76 à 2,14 (m) : 4H
de 2,32 à 3,24 (m) : 9H

7,43 (d, J = 8,5) $H_4$
8,14 (dd, J = 8,5 et 2) $H_5$ indole
9,13 (d, J = 2) $H_7$

. Isomère 9-nitro.
Spectre RMN ($CDCl_3$ 250 MHz ppm) :

1,29 (m) : 1H
de 1,83 à 3,29 (m) ~ 13H

7,35 (t, J = 8) $H_6$
8,03 (dd, J = 8 et 1) $H_7$ indole
8,75 (dd, J = 8 et 1) $H_5$

**Préparation 4 : [(16alpha) (±)] 11-diméthylamino 20,21-dinoréburnaménin-14(15H)-one utilisé au départ de l'exemple 42.**

<u>Stade A</u> **:** [(16alpha) (±)] 11-amino 20,21-dinoréburnaménin-14(15H)-one.

Un hydrogène pendant 15 heures sous 500 g de pression, 22,1 g de [(16alpha) (±)] 11-nitro 20,21-dinoréburnaménin-14(15H)-one préparé comme indiqué à la préparation 3, dans un mélange comprenant 750 cm3 d'acétate d'éthyle et 750 cm3 d'éthanol en présence de 900 mg d'oxyde de platine. On filtre, concentre à sec la phase organique, empâte le résidu dans l'éther isopropylique, sèche à 50¤C sous pression réduite et obtient 18,2 g de produit attendu. F = 172¤C puis 210¤C.
<u>Spectre RMN ($CDCl_3$ 250 MHz ppm)</u> :

1,21 (m) : 1H
1,75 à 2,03 (m) : 4H
2,25 à 3,15 (m) : 9H
3,73 (m) : $NH_2$

6,65 (dd, J = 8 et 2) $H_5$
7,16 (d, J = 8) $H_4$ indole
7,75 (d, J = 2) $H_7$

<u>Stade B</u> **:** [(16alpha) (±)] 11-diméthylamino 20,21-dinoréburnaménin-14(15H)-one.

On agite pendant 10 minutes sous atmosphère inerte 2 g de produit préparé ci-dessus en solution dans un mélange comprenant 50 cm3 d'acétonitrile et d'aldéhyde formique. On ajoute 1,33 g de cyano borohydrure de sodium, agite 30 minutes à température ambiante, ajoute goutte à goutte 0,7 cm3 d'acide acétique et poursuit l'agitation pendant 15 heures. On ajoute 100 cm3 d'eau, alcalinise par addition d'ammoniaque concentré, extrait à l'acétate d'éthyle, lave à l'eau, sèche et concentre à sec sous pression réduite. Après chromatographie sur silice (éluant : acétate d'éthyle), on obtient 1,94 g de produit pur. F = 164¤C.
<u>Spectre RMN (DMSO 250 MHz ppm)</u> :

2,99 (s) : les N-$CH_3$

6,76 (dd) $H_5$ ⎫
7,24 (d) $H_4$ ⎬ indole
7,82 (d) $H_7$ ⎭

1,24 (m) 1H ⎫
de 1,7 à 3,2 (m) ⎭ les autres protons

**Préparation 5 : [(16alpha) (±)] N-(14,15-dihydro 14-oxo 20,21-dinoréburnaménin-11-yl) acétamide utilisé au départ de l'exemple 44.**

On ajoute 1,5 cm3 de triéthylamine à 1,5 g de [(16alpha) (±)] 11-amino 20,21-dinoréburnaménin-14-(15H)-one préparé comme indiqué au stade A de la préparation 4 en solution dans 30 cm3 de tétrahydrofuranne. On ajoute goutte à goutte 0,4 cm3 de chlorure d'acétyle, agite 30 minutes à température ambiante, ajoute 100 cm3 d'eau, essore le précipité, le lave à l'eau et le sèche à 100¤C sous pression réduite. On obtient 1,75 g de produit attendu. F ≧ 260¤C.
Spectre RMN ($CDCl_3$ 250 MHz ppm) :

2,18 (s) : 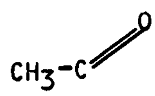
1,25 (m) : 1H
1,8 à 3,2 (m) : les autres protons

7,29 (d, J = 8,5) $H_4$ ⎫
7,72 (dd, J = 8,5 et 2) $H_5$ ⎬ indole
8,12 (d, J = 2) $H_7$ ⎭

7,64 (s) : 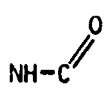

**Préparation 6 : [(16alpha) (±)] 11-éthyl 20,21-dinoréburnaménin-14(15H)-one.**

**Stade A :** [(16alpha) (±)] 11-éthényl 20,21-dinoréburnaménin-14(15H)-one.

On ajoute 2,9 cm3 de vinyltributylétain puis 100 mg de tétrakis-(triphényl phosphine)-palladium dans une solution comprenant 3 g de [(16alpha) (±)] 11-bromo 20,21-dinoréburnaménin-14(15H)-one et chauffe 24 heures au reflux. On filtre, concentre le filtrat sous pression réduite, reprend le résidu dans l'acétate d'éthyle, filtre et élimine le solvant sous pression réduite. Après chromatographie sur silice (éluant : acétate d'éthyle), on obtient 2,1 g de produit attendu. F = 164¤C.
Spectre RMN ($CDCl_3$ 300 MHz ppm) :

5,23 (d, J = 11)
5,81 (d, J = 17,5)

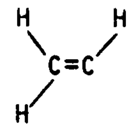

6,82 (dd, J = 11 et 17,5)
7,34 (m) $H_4$ et $H_5$ indole
8,40 (s) $H_7$

1,25 (m) 1H
1,80 à 2,1 (m) 4H les autres protons
2,30 à 3,2 (m) 9H

**Stade B :** [(16alpha) (±)] 11-éthyl 20,21-dinoréburnaménin-14(15H)-one.

On ajoute 100 mg d'hydroxyde de platine à 2 g de produit préparé au stade A en solution dans 100 cm3 d'éthanol et hydrogène sous 500 g de pression pendant 6heures. On filtre, ajoute 0,6 cm3 d'acide chlorhydrique concentré à la solution éthanolique. On agite pendant 30 minutes, essore le chlorhydrate, le reprend dans 200 cm3 d'eau, alcalinise par addition d'ammoniaque concentré, essore le précipité, le lave à l'eau, le sèche et recueille 1,35 g de produit attendu. F = 130¤C.
Spectre RMN ($CDCl_3$ 300 MHz ppm) :

1,28 (t) : $CH_3$-$CH_2$
2,76 (p) : $CH_3$-$CH_2$

7,12 (dd) $H_5$
7,32 (d) $H_4$ indole
8,21 (d) $H_7$

1,2 1H
1,8 à 3,15 (m) les autres protons

**Préparation 7 : [(16alpha) (±)] 11-éthoxy 20,21-dinoréburnaménin-14(15H)-one.**

**Stade A :** [(16alpha) (±)] 11-hydroxy 20,21-dinoréburnaménin-14(15H)-one.

On opère selon la méthode décrite Pub. Bull. Soc. Chim. Belg 88 n¤ 1-2 (1979) p.93 en utilisant au départ 560 mg de [(16alpha) (±)] 11-amino 20,21-dinoréburnaménin-14(15H)-one, 180 mg de nitrite de sodium dans 2 cm3 d'eau en présence de 4 cm3 d'acide sulfurique à 35% puis 7,5 g de nitrate cuivrique trihydraté et 290 mg d'oxyde de cuivre. On obtient 220 mg de produit attendu.

**Stade B :** [(16alpha) (±)] 11-éthoxy 20,21-dinoréburnaménin-14(15H)-one.

On ajoute 136 mg d'hydrure de sodium à une solution comprenant 800 mg de produit préparé comme au stade A et 10 cm3 de diméthylformamide, sous atmosphère inerte et agite pendant 30 minutes. On ajoute 0,25 cm3 d'iodure d'éthyle, laisse en contact 1 heure, Ajoute 200 cm3 d'eau, extrait à l'acètate d'éthyle. On lave la phase organique à l'eau, la sèche et concentre à sec. On chromatographie le résidu sur silice (éluant : acétate d'éthyle) et obtient 464 mg de produit attendu. F = 148¤C.

Spectre RMN ($CDCl_3$ 250 MHz ppm) :

1,43 (t) ) $OCH_2-CH_3$
4,10 (q) )

6,88 (dd, J = 8,5 et 2) $H_5$ )
7,27 (d, J = 8,5) $H_4$ ) indole
7,94 (d, J = 2) $H_7$ )

1,26 (m) 1H )
1,8 à 3,2 (m) ) les autres protons

**Préparation 8 : [(16alpha) (±)] 11-chloro 20,21-dinoréburnaménin-14(15H)-one.**

On refroidit à -5¤C, 6,9 g de [(16alpha) (±)] 11-amino 20,21-dinoréburnaménin-14(15H)-one en solution dans 100 cm3 d'acide chlorhydrique 6N puis ajoute goutte à goutte une solution de nitrite de sodium dans 3 cm3 d'eau. On agite 20 minutes à -5¤C, verse dans une solution de chlorure cuivreux dans 50 cm3 d'acide chlorhydrique 6N. On agite 30 minutes à 90¤C, ajoute 500 cm3 d'eau glacée, alcalinise à l'aide d'ammoniaque concentré, essore le précipité, le lave à l'eau, le sèche à 70¤C, le chromatographie sur silice (éluant : chlorure de méthylène-méthanol 96-4) et obtient 6,2 g de produit attendu. F = 205¤C.

**Exemple 61 : composition pharaceutique.**

- On a préparé des comprimés répondant à la formule suivante :

| | |
|---|---|
| . produit de l'exemple 2<br>. excipient qs pour un comprimé | 300 mg<br>350 mg |

(détail de l'excipient : talc, stéarate de magnésium, aérosil®.

**Exemple 62 : composition pharmaceutique.**

- On a préparé des comprimés répondant à la formule suivante :

| | |
|---|---|
| . produit de l'exemple 10<br>. excipient qs pour un comprimé | 300 mg<br>350 mg |

(détail de l'excipient : talc, stéarate de magnésium, aérosil®.

- idem avec produit de l'exemple 10.

**ETUDE PHARMACOLOGIOUE :**

1/. Affinité pour les récepteurs alpha2 adrénergiques :

On homogénéise dans 90 ml de sucrose 0,32M, 10 cortex prélevés sur des cerveaux de rats mâles pesant 150 g en moyenne. Après centrifugation 1 000 g du mélange homogénéisé pendant 10 minutes à 0¤C, le surnageant est centrifugé à 30 000 g pendant 10 minutes à 0¤ +4¤C. Le culot est mis en suspension dans 240 ml de tampon TrisHCl 50mM pH 7,7 et centrifugé à 30 000 g pendant 15 minutes à 0¤ +4¤C. Le nouveau culot obtenu est mis en suspension dans 480 ml de tampon $NaKPO_4$ pH 7,4 50mM.

On fait ensuite incuber pendant 45 minutes à 25¤C, 2 ml de suspension en présence de $^3H$ rauwolscine à la concentration 0,15 nM :

i) seule,

ii) avec des concentrations croissantes du produit à tester ou,

iii) pour déterminer la fixation non spécifique,

avec de la phentolamine non radioactive à la concentration $10^{-5}$ M.

Les suspensions incubées sont filtrées sur Whatman GF/C et les filtres sont lavés par trois fois 5 ml de tampon $NaKPO_4$ pH 7,4 à 0¤C. La radioactivité des filtres est mesurée par scintillation liquide.

L'affinité du produit testé pour les récepteurs alpha2 adrénergiques est donnée relativement à la phentolamine comme produit de référence.

CD = concentration de phentolamine inhibant 50 % de la fixation spécifique de la $^3H$ rauwolscine ;

CX = concentration du produit à tester inhibant 50 % de la fixation spécifique de la $^3H$ rauwolscine.

L'affinité relative est donnée par la relation : $ARL = 100 \frac{CD}{CX}$

**Résultats :**

| produits des exemples | ARL |
|---|---|
| 2 | 110 |
| 4 | 35 |
| 5 | 55 |
| 10 | 160 |
| 12 | 39 |
| 16 | 136 |
| 18 | 39 |

2/. Anoxie asphyxique :

L'épreuve est réalisée chez le rat mâle Spragne Dawley (Charles River) anesthésié à l'éther éthylique, immobilisé (tubécurarine 1 mg/Kg I.V.) et ventilés artificiellement à l'air. On enregistre l'électrocorticogramme (ECoG) et la pression artérielle. La température rectale est maintenue autour de 36¤C et la capnie entre 35 et 40 torr. Les produits sont administrés à la dose de 10 mg/kg par voie I.V. sous un volume de 1 ml/Kg 3 mn avant l'asphyxie obtenue par arrêt de la respiration artificielle. On mesure le temps de latence de disparition de l'ECoG.

**Résultats :**

| produit de l'exemple | % de variation du temps de latence de disparition de l'ECoG après asphyxie |
|---|---|
| 2 | +43 |
| 5 | +26 |
| 8 | +37 |
| 10 | +47 |
| 12 | +18 |
| 16 | +22 |
| 21 | +30 |
| 22 | +18 |
| 24 | +35 |
| 25 | +20 |
| 28 | +35 |
| 29 | +25 |

3/. Test d'anoxie hypobare chez la souris.

Il consiste à mesurer sur une durée maximale de 3 minutes le temps de survie de souris placées dans une enceinte de 2 litres dans laquelle on réalise une dépression de 600 mmHg. On utilise des souris à jeun depuis 6 heures. Les produits sont administrés à la dose de 10 mg/kg par voie i.p. sous un volume de 0,2 ml/10 g 60 minutes avant l'épreuve.

On note l'augmentation du temps de survie, exprimé en pourcentage, des animaux traités par rapport aux animaux témoins, soumis aux mêmes conditions.

On a obtenu les résultats suivants :

| Produit de l'exemple | % d'augmentation du temps de survie |
|---|---|
| 39 | 14 |
| 40 | 22 |
| 41 | 13 |

**Effet arti-amnésiant dans un évitement passif.**

Des rats sont placés individuellement dans le compartiment éclairé d'une boîte à deux compartiments, l'autre étant obscure. Ils se réfugient spontanément dans le compartiment obscur et dès leur entrée, les rats reçoivent un choc électrique (1mA/5sec) par le plancher grillagé. Les animaux sont alors divisés en 3 groupes : le 1er groupe (témoin) ne subit pas d'autre manipulation. Dans le 2ème groupe, le choc électrique est immédiatement suivi de l'application d'un électrochoc amnésiant (60mA, 0,6ms, 0,6s) groupe témoin électrochoc). Le 3ème groupe est identique au 2ème, mais l'électrochoc est immédiatement suivi de l'administration du composé à tester (groupe traité). Vingt-quatre heures plus tard, les animaux sont replacés dans le compartiment éclairé de la boîte et on mesure le temps de latence d'entrée dans le compartiment sombre (jusqu'à 300 sec maxi). Chez les témoins, ce temps est voisin de 300 sec. Les témoins électrochoc pénètrent au contraire beaucoup plus rapidement dans le compartiment sombre (effet amnésiant). Les produits à effet anti-amnésiant augmentent la latence d'entrée et tendent à la ramener à une valeur comparable à celle des témoins sans électrochoc.

Sur ce test le composé de l'exemple 10 réverse l'efet anti-amnésiant d'un électrochoc à la dose de 2 mg/kg par voie orale.

4/. Test d'alternance spontanée après lésion septale cholinergique.

Le comportement d'alternance spontanée est une caractéristique essentielle du répertoire comportemental du rat, qui, mis en situation de choix entre un stimulus familier et un stimulus nouveau, choisira ce dernier. Ainsi, dans le cas d'un labyrinthe en Y, l'animal choisira de préférence un bras non encore exploré par rapport à un bras qu'il vient de visiter (alternance spontanée). Cette faculté de discrimination entre le "familier" et le "nouveau" implique la mise en jeu d'une forme de mémoire, la Mémoire à Court Terme ou Mémoire de Travail. Les performances d'alternance spontanée permettent donc une évaluation de la capacité de ce type de mémoire.

La méthode utilisée consiste à créer un déficit mnésique chez le rat, par lésion du système cholinergique septohippocampique et, à rechercher si le produit testé est suceptible de réverser ce déficit. Dans le cas présent, ce déficit est évalué dans un comportement d'alternance spontanée ; l'animal lésé a des performances d'alternance voisines de 50%, car il effectue ses choix par hasard.

Chaque expérience comporte un groupe témoin-véhicule, un groupe lésé-véhicule et un ou deux groupes lésés recevant le produit testé. Pendant 4 jours, chaque rat est soumis à une séance par jour, comprenant quatre essais eux-mêmes divisés en deux parties : un choix forcé et un choix libre. Le produit est administré par voie intrapéritonéale (i.p.) 30 minutes avant chaque séance.

Le produit de l'exemple 10 réverse le déficit mnésique provoqué par la lésion septale cholinergique dans une gamme de doses allant de 1 à 10 mg/kg i.p.

5/. Test d'activité antidépressive.

Les essais sont effectués sur des lots de 5 rats Sprague Dawley. Les animaux naïfs, sont placés pendant 15 minutes dans un cylindre vertical en plexiglass (diamètre : 18 cm, hauteur : 40 cm) contenant de l'eau à 25¤C sur une hauteur de 15 cm (épreuve initiale de nage). Ils sont ensuite séchés pendant 15 minutes dans une enceinte chauffée à 32¤C, 24 heures plus tard, ils sont replacès dans le cylindre rempli d'eau et la durée totale des périodes d'immobilité est mesurée pendant 5 minutes.

Le composé est administré i.p. successivement 24, 5 et 1/2 heure avant le test. La première administration a lieu immédiatement après l'épreuve initiale de nage, juste avant de replacer les animaux dans leur boîte d'élevage.

Les moyennes des groupes traités sont comparées à celles du groupe témoin par le test de Dunnett.

**Résultats :**

| Produit de l'exemple | $DA_{50}$ mg/kg |
|---|---|
| 10 | 13 |

**Revendications**

**Revendications pour les Etats contractants suivants : AT, BE, CH, DE, FR, GB, IT, LI, LU, NL, SE**

**1.** Composés de formule (I) :

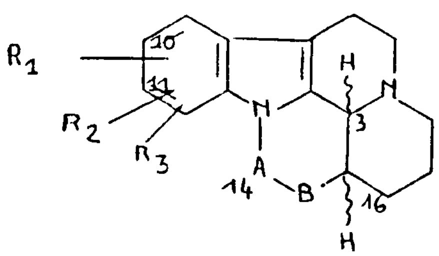

(I)

dans laquelle $R_1$, $R_2$ et $R_3$, identiques ou différents, représentent un atome d'hydrogène, un atome d'halogène, un radical alcoyle ou alcoxy renfermant de 1 à 5 atomes de carbone, un radical hyoroxy, trifluorométhyle, nitro, amino, alcoylamino, dialcoylamino dans lequel le radical alcoyle renferme de 1 à

5 atomes de carbone, un radical acylamino dans lequel le radical acyle représente le reste d'un acide aliphatique renfermant jusqu'à 6 atomes de carbone, $R_1$, $R_2$ et $R_3$ ne pouvant pas représenter simultanément un atome d'hydrogène et dans laquelle le groupement :

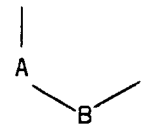

représente soit :

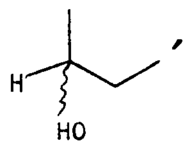

soit :

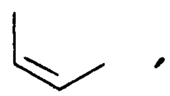

ou soit :

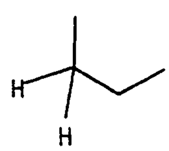

lesdits produits de formule (I) étant sous toutes les formes isomères possibles racémiques ou optiquement actives ainsi que les sels d'addition avec les acides minéraux ou organiques desdits produits de formule (I).

**2.** Composés de formule (I), tels que définis à la revendication 1, caractérisés en ce que $R_1$, $R_2$ et $R_3$ identiques ou différents représentent un atome d'hydrogène ou un radical méthyle, éthyle, méthoxy ou éthoxy, un atome de chlore, un radical hydroxy, trifluorométhyle ou nitro, sous toutes les formes isomères possibles, racémiques ou optiquement actives, ainsi que les sels d'addition avec les acides minéraux ou organiques.

**3.** Composés de formule (I), tels que définis à la revendication 1 ou 2, caractérisés en ce que l'un des trois substituants $R_1$ ou $R_2$ ou $R_3$ représente en position 10 ou 11 un radical méthyle, éthyle, méthoxy ou éthoxy, un atome de chlore, un radical hydroxy, trifluorométhyle ou nitro, les deux autres substituants représentant un atome d'hydrogène, sous toutes les formes isomères possibles, racémiques ou optiquement actives, ainsi que les sels d'addition avec les acides minéraux ou organiques.

**4.** Composés de formule (I), tels que définis à l'une des revendications 1 ou 2, caractérisés en ce que deux des trois substituants $R_1$, $R_2$ ou $R_3$ représentent en position 9, 10 ou 11 un atome de chlore, un radical méthyle, éthyle, méthoxy ou éthoxy, le troisième substituant représentant un atome d'hydrogène ou en ce que $R_1$, $R_2$ et $R_3$ représentent tous trois dans ces positions un atome de chlore ou un radical méthyle, éthyle, méthoxy ou éthoxy sous toutes les formes isomères possibles, racémiques ou optiquement actives, ainsi que les sels d'addition avec les acides minéraux ou organiques.

5. Composés de formule (I), tels que définis à l'une des revendications 1 à 4, caractérisés en ce que l'atome d'hydrogène en position 3 et l'atome d'hydrogène en position 16 sont trans, sous toutes les formes isomères posssibles racémiques ou optiquement actives ainsi que les sels d'addition avec les acides minéraux ou organiques.

6. Composés de formule (I), tels que définis à l'une des revendications 1 à 5, caractérisés en ce que le groupement

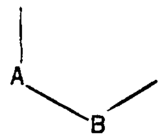

représente soit

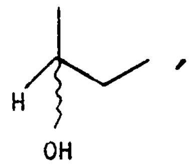

soit

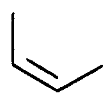

sous toutes les formes isomères possibles, racémiques ou optiquement actives, ainsi que les sels d'addition avec les acides minéraux ou organiques.

7. Composés de formule (I), dont les noms suivent :
   - [(+)(14alpha, 16alpha)] 14,15-dihydro 10-méthoxy 20,21 -dinoréburnaménin-14-ol,
   - ((±) (14alpha, 16alpha)] 14,15-dihydro 11-méthyl 20,21-dinoréburnaménin-14-ol,
   - [(±) (16alpha)] 11-chloro 20,21-dinoréburnaménine,
   - [(±) (16alpha)] 11-méthoxy 20,21-dinoréburnaménine,
   - [(±) (16alpha)] 11-méthyl 20,21-dinoréburnaménine,

   ainsi que leurs sels d'addition avec les acides minéraux ou organiques.

8. Procédé de préparation des composés de formule (I) telle que définie à la revendication 1, caractérisé en ce que l'on réduit un composé de formule (II):

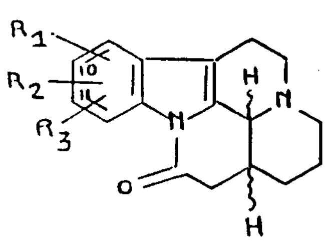

(II)

dans laquelle $R_1$, $R_2$ et $R_3$ ont les significations déjà indiquées pour obtenir un composé de formule ($I_A$) correspondant à un produit de formule (I) dans laquelle :

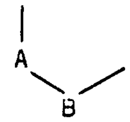

représente

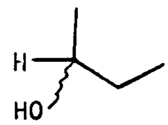

ledit composé de formule ($I_A$) étant, si désiré, déshydraté pour obtenir un composé correspondant de formule ($I_B$), représentant un composé de formule (I) dans laquelle :

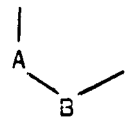

représente

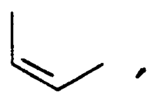

ledit composé de formule ($I_B$) étant, si désiré, réduit en composé correspondant de formule ($I_C$), représentant un composé de formule (I) dans laquelle :

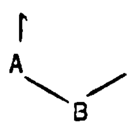

représente

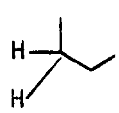

et traite si désiré tous les produits de formule (I) obtenus par un acide minéral ou organique pour en former le sel.

**9.** A titre de médicaments, les composés tels que définis par la formule (I) de la revendication 1, lesdits produits de formule (I) étant sous toutes les formes isomères possibles, racémiques ou optiquement actives, ainsi que les sels d'addition avec les acides minéraux ou organiques pharmaceutiquement acceptables.

**10.** A titre de médicaments, les composés tels que définis par les revendications 2 à 6, sous toutes les formes isomères possibles, racémiques ou optiquement actives, ainsi que les sels d'addition avec les

acides minéraux ou organiques pharmaceutiquement acceptables.

**11.** A titre de médicaments, les composés tels que définis par la revendication 7, ainsi que leurs sels d'addition avec les acides minéraux ou organiques pharmaceutiquement acceptables.

**12.** Les compositions pharmaceutiques contenant à titre de principe actif, l'un au moins des médicaments tels que définis à l'une quelconque des revendications 9 à 11.

**13.** Les produits de formule (II) tels que définis à la revendication 8 à l'exception des produits de formule ($II_A$) :

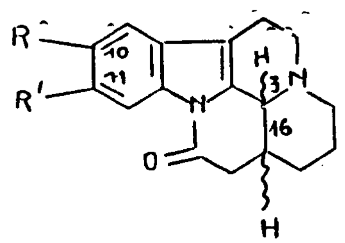

($II_A$)

pour lesquels R et R' identiques ou différents représentent un radical hydroxy ou méthoxy.

**Revendications pour l'Etat contractant suivant : ES**

**1.** Procédé pour préparer des composés de formule (I) :

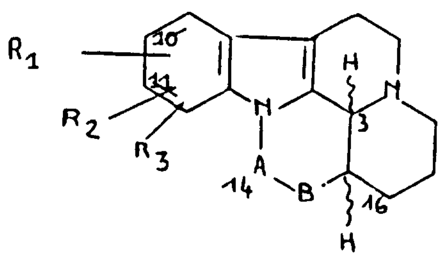

(I)

dans laquelle $R_1$, $R_2$ et $R_3$, identiques ou différents, représentent un atome d'hydrogène, un atome d'halogène, un radical alcoyle ou alcoxy renfermant de 1 à 5 atomes de carbone, un radical hydroxy, trifluorométhyle, nitro, amino, alcoylamino, dialcoylamino dans lequel le radical alcoyle renferme de 1 à 5 atomes de carbone, un radical acylamino dans lequel le radical acyle représente le reste d'un acide aliphatique renfermant jusqu'à 6 atomes de carbone, $R_1$, $R_2$ et $R_3$ ne pouvant pas représenter simultanément un atome d'hydrogène et dans laquelle le groupement :

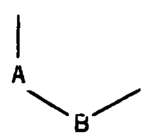

représente soit :

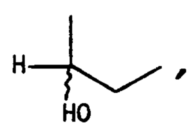

soit :

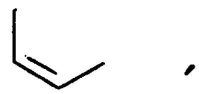

ou soit :

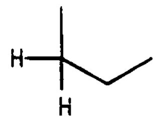

lesdits produits de formule (I) étant sous toutes les formes isomères possibles racémiques ou optiquement actives ainsi que les sels d'addition avec les acides minéraux ou organiques desdits produits de formule (I), caractérisé en ce que l'on réduit un composé de formule (II) :

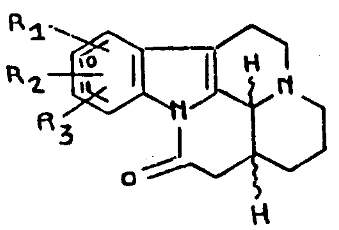

(II)

dans laquelle $R_1$, $R_2$ et $R_3$ ont les significations déjà indiquées pour obtenir un composé de formule ($I_A$) correspondant à un produit de formule (I) dans laquelle :

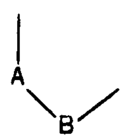

représente

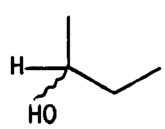

ledit composé de formule ($I_A$) étant, si désiré, déshydraté pour obtenir un composé correspondant de formule ($I_B$), représentant un composé de formule (I) dans laquelle :

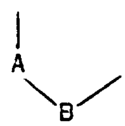

représente

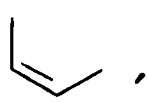

ledit composé de formule ($I_B$) étant, si désiré, réduit en composé correspondant de formule ($I_C$), représentant un composé de formule (I) dans laquelle :

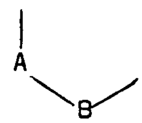

représente

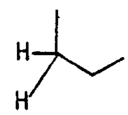

et traite si désiré tous les produits de formule (I) obtenus par un acide minéral ou organique pour en former le sel.

2. Procédé selon la revendication 1, caractérisé en ce que l'on utilise au départ un produit de formule (II) dans laquelle $R_1$, $R_2$ et $R_3$ identiques ou différents représentent un atome d'hydrogène ou un radical méthyle, éthyle, méthoxy ou éthoxy, un atome de chlore, un radical hydroxy, trifluorométhyle ou nitro.

3. Procédé selon la revendication 1 ou 2, caractérisé en ce que l'on utilise au départ un produit de formule (II) dans laquelle l'un des trois substituants $R_1$ ou $R_2$ ou $R_3$ représente en position 10 ou 11 un radical méthyle, éthyle, méthoxy ou éthoxy, un atome de chlore, un radical hydroxy, trifluorométhyle ou nitro, les deux autres substituants représentant un atome d'hydrogène.

4. Procédé selon l'une quelconque des revendications 1 ou 2, caractérisé en ce que l'on utilise au départ un produit de formule (II) dans laquelle deux des trois substituants $R_1$, $R_2$ ou $R_3$ représentent en position 9, 10 ou 11 un atome de chlore, un radical méthyle, éthyle, méthoxy ou éthoxy, le troisième substituant représentant un atome d'hydrogène ou en ce que $R_1$, $R_2$ et $R_3$ représentent tous trois dans ces positions un atome de chlore ou un radical méthyle, éthyle, méthoxy ou éthoxy.

5. Procédé selon l'une quelconque des revendications 1 à 4, caractérisé en ce que l'on utilise au départ un produit de formule (II) dans laquelle l'atome d'hydrogène en position 3 et l'atome d'hydrogène en position 16 sont trans.

6. Procédé selon l'une quelconque des revendications 1 à 5, caractérisé en ce que l'on soumet un produit de formule (II) à l'action d'un agent réducteur approprié pour obtenir un produit correspondant de formule (I) dans laquelle

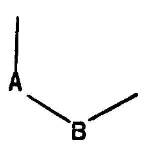

représente

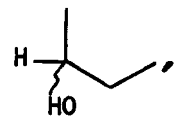

que le cas échéant, l'on soumet à l'action d'un agent déshydratant approprié, pour obtenir un produit

correspondant de formule (I) dans laquelle

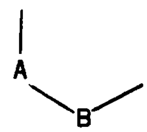

représente

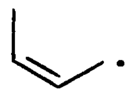

7. Procédé selon la revendication 1, caractérisé en ce que l'on prépare l'un quelconque des produits dont les noms suivent :
   - [(+)(14alpha, 16alpha)] 14,15-dihydro 10-méthoxy 20,21-dinoréburnaménin-14-ol,
   - [(±) (14alpha, 16alpha)] 14,15-dihydro 11-méthyl 20,21-dinoréburnaménin-14-ol,
   - [(±) (16alpha)] 11-chloro 20,21-dinoréburnaménine,
   - [(±) (16alpha)] 11-méthoxy 20,21-dinoréburnaménine,
   - [(±) (16alpha)] 11-méthyl 20,21-dinoréburnaménine,

   ainsi que leurs sels d'addition avec les acides minéraux ou organiques.

**Revendications pour l'Etat contractant suivant : GR**

1. Procédé pour préparer des composés de formule (I) :

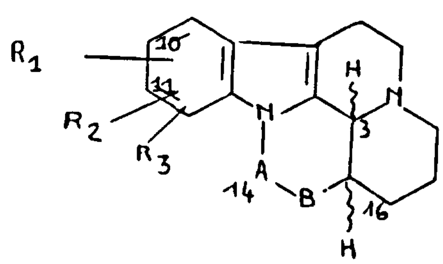

(I)

dans laquelle $R_1$, $R_2$ et $R_3$, identiques ou différents, représentent un atome d'hydrogène, un atome d'halogène, un radical alcoyle ou alcoxy renfermant de 1 à 5 atomes de carbone, un radical hydroxy, trifluorométhyle, nitro, amino, alcoylamino, dialcoylamino dans lequel le radical alcoyle renferme de 1 à 5 atomes de carbone, un radical acylamino dans lequel le radical acyle représente le reste d'un acide aliphatique renfermant jusqu'à 6 atomes de carbone, $R_1$, $R_2$ et $R_3$ ne pouvant pas représenter simultanément un atome d'hydrogène et dans laquelle le groupement :

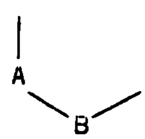

représente soit :

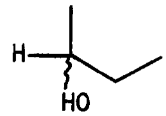 ,

soit :

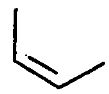 ,

ou soit :

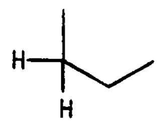

lesdits produits de formule (I) étant sous toutes les formes isomères possibles racémiques ou optiquement actives ainsi que les sels d'addition avec les acides minéraux ou organiques desdits produits de formule (I), caractérisé en ce que l'on réduit un composé de formule (II) :

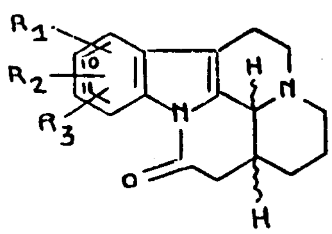

(II)

dans laquelle $R_1$, $R_2$ et $R_3$ ont les significations déjà indiquées pour obtenir un composé de formule ($I_A$) correspondant à un produit de formule (I) dans laquelle :

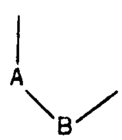

représente

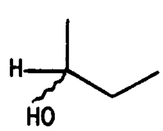

ledit composé de formule ($I_A$) étant, si désiré, déshydraté pour obtenir un composé correspondant de formule ($I_B$), représentant un composé de formule (I) dans laquelle :

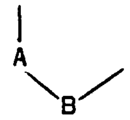

représente

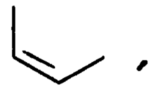

ledit composé de formule ($I_B$) étant, si désiré, réduit en composé correspondant de formule ($I_C$), représentant un composé de formule (I) dans laquelle :

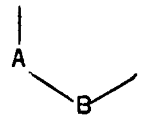

représente

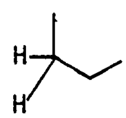

et traite si désiré tous les produits de formule (I) obtenus par un acide minéral ou organique pour en former le sel.

2. Procédé selon la revendication 1, caractérisé en ce que l'on utilise au départ un produit de formule (II) dans laquelle $R_1$, $R_2$ et $R_3$ identiques ou différents représentent un atome d'hydrogène ou un radical méthyle, éthyle, méthoxy ou éthoxy, un atome de chlore, un radical hydroxy, trifluorométhyle ou nitro.

3. Procédé selon la revendication 1 ou 2, caractérisé en ce que l'on utilise au départ un produit de formule (II) dans laquelle l'un des trois substituants $R_1$ ou $R_2$ ou $R_3$ représente en position 10 ou 11 un radical méthyle, éthyle, méthoxy ou éthoxy, un atome de chlore, un radical hydroxy, trifluorométhyle ou nitro, les deux autres substituants représentant un atome d'hydrogène.

4. Procédé selon l'une quelconque des revendications 1 ou 2, caractérisé en ce que l'on utilise au départ un produit de formule (II) dans laquelle deux des trois substituants $R_1$, $R_2$ ou $R_3$ représentent en position 9, 10 ou 11 un atome de chlore, un radical méthyle, éthyle, méthoxy ou éthoxy, le troisième substituant représentant un atome d'hydrogène ou en ce que $R_1$, $R_2$ et $R_3$ représentent tous trois dans ces positions un atome de chlore ou un radical méthyle, éthyle, méthoxy ou éthoxy.

5. Procédé selon l'une quelconque des revendications 1 à 4, caractérisé en ce que l'on utilise au départ un produit de formule (II) dans laquelle l'atome d'hydrogène en position 3 et l'atome d'hydrogène en position 16 sont trans.

6. Procédé selon l'une quelconque des revendications 1 à 5, caractérisé en ce que l'on soumet un produit de formule (II) à l'action d'un agent réducteur approprié pour obtenir un produit correspondant de formule (I) dans laquelle

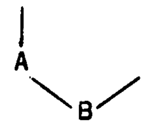

représente

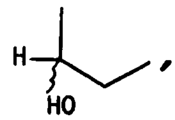

que le cas échéant, l'on soumet à l'action d'un agent déshydratant approprié, pour obtenir un produit correspondant de formule (I) dans laquelle

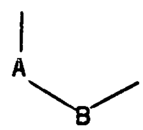

représente

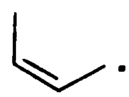

**7.** Procédé selon la revendication 1, caractérisé en ce que l'on prépare l'un quelconque des produits dont les noms suivent :
- [(+)(14alpha, 16alpha)] 14,15-dihydro 10-méthoxy 20,21-dinoréburnaménin-14-ol,
- [(±) (14alpha, 16alpha)] 14,15-dihydro 11-méthyl 20,21-dinoréburnaménin-14-ol,
- [(±) (16alpha)] 11-chloro 20,21-dinoréburnaménine,
- [(±) (16alpha)] 11-méthoxy 20,21-dinoréburnaménine,
- [(±) (16alpha)] 11-méthyl 20,21-dinoréburnaménine,

ainsi que leurs sels d'addition avec les acides minéraux ou organiques.

**8.** Procédé de préparation de compositions pharmaceutiques, caractérisé en ce que l'on met à titre de principe actif l'un au moins des composés de formule (I), telle que définie à la revendication 1 ou l'un au moins de leurs sels pharmaceutiquement acceptables, sous une forme destinée à cet usage.

**9.** Procédé selon la revendication 8, caractérisé en ce que le composé de formule (I) est choisi parmi les produits dont les noms suivent :
- [(+)(14alpha, 16alpha)] 14,15-dihydro 10-méthoxy 20,21-dinoréburnaménin-14-ol,
- [(±) (14alpha, 16alpha)] 14,15-dihydro 11-méthyl 20,21-dinoréburnaménin-14-ol,
- [(±) (16alpha)] 11-chloro 20,21-dinoréburnaménine,
- [(+) (16alpha)] 11-méthoxy 20,21-dinoréburnaménine,
- [(±) (16alpha)] 11-méthyl 20,21-dinoréburnaménine,

ainsi que leurs sels d'addition avec les acides minéraux ou organiques.

**10.** Les produits de formule (II) tels que définis à la revendication 1 à l'exception des produits de formule ($II_A$) :

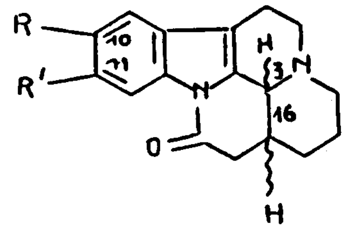

$(II_A)$

pour lesquels R et R' identiques ou différents représentent un radical hydroxy ou méthoxy.

## Claims

## Claims for the following Contracting States : AT, BE, CH, DE, FR, GB, IT, LI, LU, NL, SE

**1.** The compounds of formula (I):

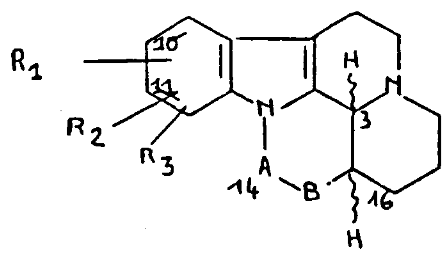

(I)

in which $R_1$, $R_2$ and $R_3$, identical or different, represent a hydrogen atom, a halogen atom, an alkyl or alkoxy radical containing 1 to 5 carbon atoms, a hydroxy, trifluoromethyl, nitro, amino, alkylamino radical, a dialkylamino radical in which the alkyl radical contains 1 to 5 carbon atoms, an acylamino radical in which the acyl radical represents the remainder of an aliphatic acid containing up to 6 carbon atoms, $R_1$, $R_2$ and $R_3$ cannot simultaneously represent a hydrogen atom and in which the group:

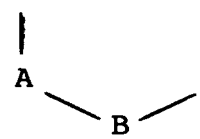

represents either:

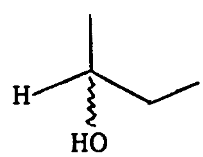

,

or:

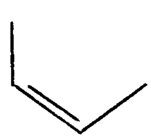

,

or:

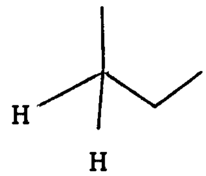

the said products of formula (I) being in all possible racemic or optically active isomer forms as well as the addition salts with mineral or organic acids of said products of formula (I).

2. The compounds of formula (I) as defined in claim 1, characterized in that $R_1$, $R_2$ and $R_3$, identical or different, represent a hydrogen atom or a methyl, ethyl, methoxy or ethoxy radical, a chlorine atom, a hydroxy, trifluoromethyl or nitro radical, in all possible racemic or optically active isomer forms, as well as the addition salts with mineral or organic acids

3. The compounds of formula (I) as defined in claim 1 or 2, characterized in that one of the three substituents $R_1$ or $R_2$ or $R_3$ represents in position 10 or 11 a methyl, ethyl, methoxy or ethoxy radical, a chlorine atom, a hydroxy, trifluoromethyl or nitro radical, the other two substituents representing a hydrogen atom, in all possible racemic or optically active isomer forms, as well as the addition salts with mineral or organic acids.

4. The compounds of formula (I) as defined in one of claims 1 or 2, characterized in that two of the three substituents $R_1$, $R_2$ or $R_3$ represent in position 9, 10 or 11 a chlorine atom, a methyl, ethyl, methoxy or ethoxy radical, the third substituent representing a hydrogen atom, or in that all three of $R_1$, $R_2$ and $R_3$ represent in these positions a chlorine atom or a methyl, ethyl, methoxy or ethoxy radical, in all possible racemic or optically active isomer forms as well as the addition salts with mineral or organic acids.

5. The compounds of formula (I) as defined in one of claims 1 to 4, characterized in that the hydrogen in position 3 and the hydrogen atom in position 16 are trans, in all possible racemic or optically active isomer forms as well as the addition salts with mineral or organic acids.

6. The compounds of formula (I) as defined in one of claims 1 to 5, characterized in that the group

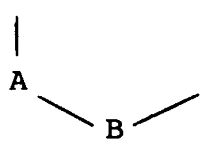

represents either

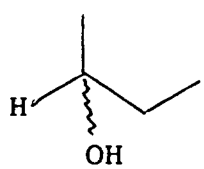

,

or

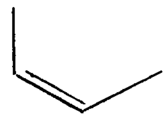

in all possible racemic or optically active isomer forms, as well as the addition salts with mineral or organic acids

**7.** The compounds of formula (I), the names of which follow:
- [(+)(14alpha, 16alpha)] 14,15-dihydro 10-methoxy 20,21-dinoreburnamenin-14-ol,
- [(±)(14alpha, 16alpha)] 14,15-dihydro 11-methyl 20,21-dinoreburnamenin-14-ol,
- [(±)(16alpha)] 11-chloro 20,21-dinoreburnamenine,
- [(±)(16alpha)] 11-methoxy 20,21-dinoreburnamenine,
- [(±)(16alpha)] 11-methyl 20,21-dinoreburnamenine,

as well as their addition salts with mineral or organic acids.

**8.** Preparation process for the compounds of formula (I) as defined in claim 1, characterized in that a compound of formula (II):

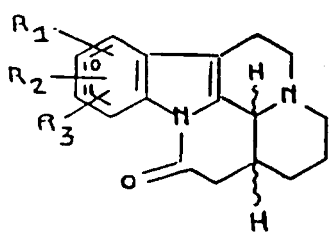

(II)

in which $R_1$, $R_2$ and $R_3$ have the meanings already indicated, is reduced in order to obtain a compound of formula ($I_A$) corresponding to a compound of formula (I) in which:

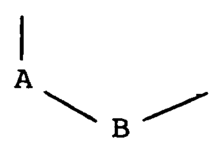

represents

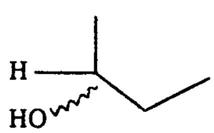

the said compound of formula ($I_A$) being, if desired, dehydrated in order to obtain a corresponding compound of formula ($I_B$), representing a compound of formula (I) in which:

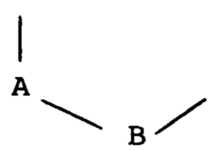

represents

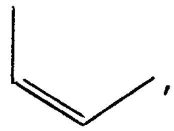

the said compound of formula ($I_B$) being, if desired, reduced into a corresponding compound of formula ($I_C$), representing a compound of formula (I) in which:

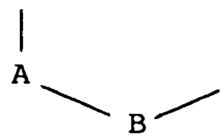

represents

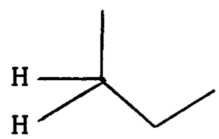

and if desired all the products of formula (I) obtained are treated with a mineral or organic acid in order to form the salt.

**9.** As medicaments, the compounds as defined by formula (I) of claim 1, the said products of formula (I) being in all possible racemic or optically active isomer forms, as well as the addition salts with pharmaceutically acceptable mineral or organic acids.

**10.** As medicaments, the compounds as defined by claims 2 to 6, in all possible racemic or optically active isomer forms, as well as the addition salts with pharmaceutically acceptable mineral or organic acids.

**11.** As medicaments, the compounds as defined by claim 7, as well as their addition salts with pharmaceutically acceptable mineral or organic acids.

**12.** The pharmaceutical compositions containing at least one of the medicaments as defined in any one of claims 9 to 11 as active ingredient.

**13.** The products of formula (II) as defined in claim 8 with the exception of the products of formula ($II_A$):

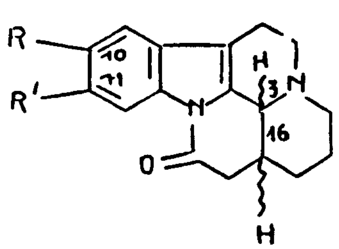

($II_A$)

for which R and R', identical or different, represent a hydroxy or methoxy radical.

**Claims for the following Contracting State : ES**

**1.** Process for the preparation of compounds of formula (I):

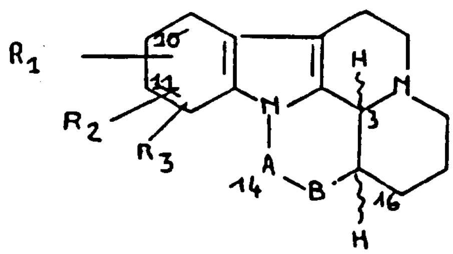

(I)

in which $R_1$, $R_2$ and $R_3$, identical or different, represent a hydrogen atom, a halogen atom, an alkyl or alkoxy radical containing 1 to 5 carbon atoms, a hydroxy, trifluoromethyl, nitro, amino, alkylamino radical, a dialkylamino radical in which the alkyl radical contains 1 to 5 carbon atoms, an acylamino radical in which the acyl radical represents the remainder of an aliphatic acid containing up to 6 carbon atoms, $R_1$, $R_2$ and $R_3$ cannot simultaneously represent a hydrogen atom and in which the group:

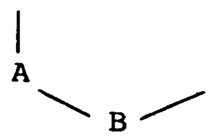

represents either:

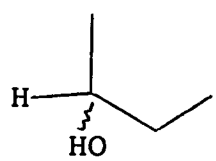

,

or:

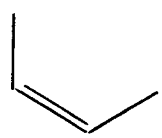

,

or:

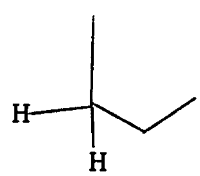

the said products of formula (I) being in all possible racemic or optically active isomer forms, as well as the addition salts with mineral or organic acids of said products of formula (I), characterized in that a compound of formula (II):

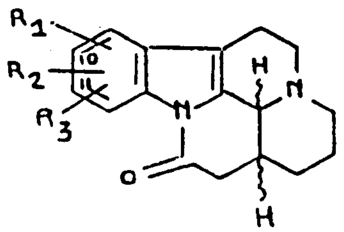

(II)

in which $R_1$, $R_2$ and $R_3$ have the meanings already indicated, is reduced in order to obtain a compound of formula ($I_A$) corresponding to a product of formula (I) in which:

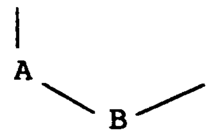

represents

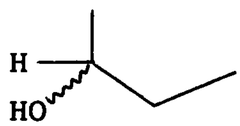

the said compound of formula ($I_A$) being, if desired, dehydrated in order to obtain a corresponding compound of formula ($I_B$), representing a compound of formula (I) in which:

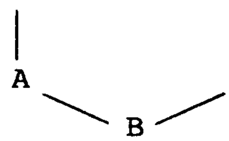

represents

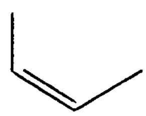

,

the said compound of formula ($I_B$) being, if desired, reduced into a corresponding compound of formula ($I_C$), representing a compound of formula (I) in which:

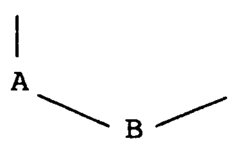

represents

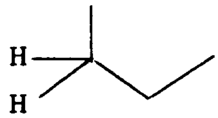

and if desired all the products of formula (I) obtained are treated with a mineral or organic acid in order to form the salt.

2. Process according to claim 1, characterized in that a product of formula (II) is used at the start in which $R_1$, $R_2$ and $R_3$, identical or different, represent a hydrogen atom or a methyl, ethyl, methoxy or ethoxy radical, a chlorine atom, a hydroxy, trifluoromethyl or nitro radical.

3. Process according to claim 1 or 2, characterized in that a product of formula (II) is used at the start in which one of the three substituents $R_1$ or $R_2$ or $R_3$ represents in position 10 or 11 a methyl, ethyl, methoxy or ethoxy radical, a chlorine atom, a hydroxy, trifluoromethyl or nitro radical, the other two substituents representing a hydrogen atom.

4. Process according to any one of claims 1 or 2, characterized in that a product of formula (II) is used at the start in which two of the three substituents $R_1$, $R_2$ or $R_3$ represent in position 9, 10 or 11 a chlorine atom, a methyl, ethyl, methoxy or ethoxy radical, the third substituent representing a hydrogen atom, or in that all three of $R_1$, $R_2$ and $R_3$ represent in these positions a chlorine atom or a methyl, ethyl, methoxy or ethoxy radical.

5. Process according to any one of claims 1 to 4, characterized in that a product of formula (II) is used at the start in which the hydrogen atom in position 3 and the hydrogen atom in position 16 are trans.

6. Process according to any one of claims 1 to 5, characterized in that a product of formula (II) is subjected to the action of an appropriate reducing agent in order to obtain a corresponding product of formula (I) in which

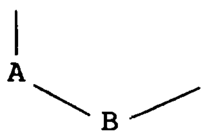

represents

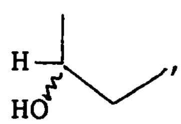

which if appropriate, is subjected to the action of an appropriate dehydration agent, in order to obtain a corresponding product of formula (I) in which

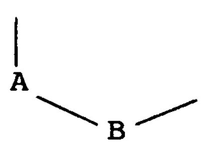

represents

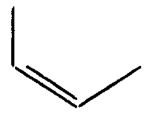

.

7. Process according to claim 1, characterized in that any one of the products whose names follow are prepared:
   - [(+)(14alpha, 16alpha)] 14,15-dihydro 10-methoxy 20,21-dinoreburnamenin-14-ol,
   - [(±)(14alpha, 16alpha)] 14,15-dihydro 11-methyl 20,21-dinoreburnamenin-14-ol,
   - [(±)(16alpha)] 11-chloro 20,21-dinoreburnamenine,
   - [(±)(16alpha)] 11-methoxy 20,21-dinoreburnamenine,
   - [(±)(16alpha)] 11-methyl 20,21-dinoreburnamenine,

   as well as their addition salts with mineral or organic acids.

**Claims for the following Contracting State : GR**

1. Process for the preparation of compounds of formula (I):

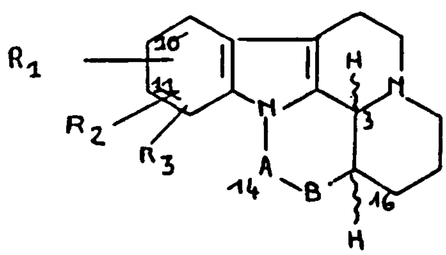

(I)

in which $R_1$, $R_2$ and $R_3$, identical or different, represent a hydrogen atom, a halogen atom, an alkyl or alkoxy radical containing 1 to 5 carbon atoms, a hydroxy, trifluoromethyl, nitro, amino, alkylamino radical, a dialkylamino radical in which the alkyl radical contains 1 to 5 carbon atoms, an acylamino radical in which the acyl radical represents the remainder of an aliphatic acid containing up to 6 carbon atoms, $R_1$, $R_2$ and $R_3$ cannot simultaneously represent a hydrogen atom and in which the group:

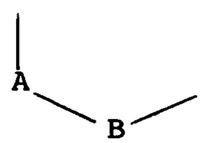

represents either:

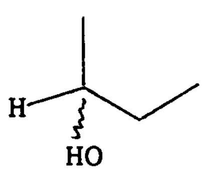

,

or:

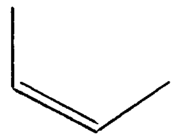
,

or:

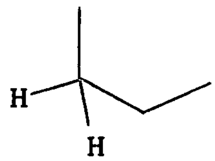

the said products of formula (I) being in all possible racemic or optically active isomer forms as well as the addition salts with mineral or organic acids of said products of formula (I), characterized in that a compound of formula (II):

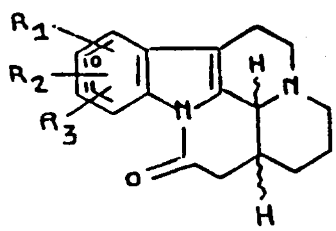
(II)

in which $R_1$, $R_2$ and $R_3$ have the meanings already indicated is reduced in order to obtain a compound of formula ($I_A$) corresponding to a compound of formula (I) in which:

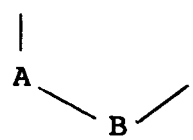

represents

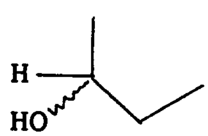

the said compound of formula ($I_A$) being, if desired, dehydrated in order to obtain a corresponding compound of formula ($I_B$), representing a compound of formula (I) in which:

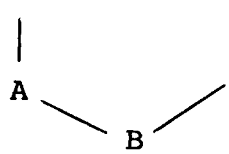

represents

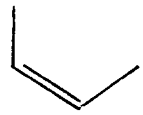 ,

the said compound of formula ($I_B$) being, if desired, reduced into a corresponding compound of formula ($I_C$), representing a compound of formula (I) in which:

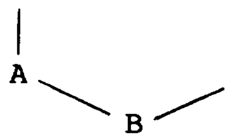

represents

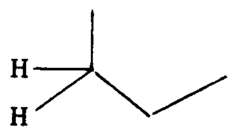

and if desired all the products of formula (I) obtained are treated with a mineral or organic acid in order to form the salt.

**2.** Process according to claim 1, characterized in that a product of formula (II) is used at the start in which $R_1$, $R_2$ and $R_3$, identical or different, represent a hydrogen atom or a methyl, ethyl, methoxy or ethoxy radical, a chlorine atom, a hydroxy, trifluoromethyl or nitro radical.

**3.** Process according to claim 1 or 2, characterized in that a product of formula (II) is used at the start in which one of the three substituents $R_1$ or $R_2$ or $R_3$ represents in position 10 or 11 a methyl, ethyl, methoxy or ethoxy radical, a chlorine atom, a hydroxy, trifluoromethyl or nitro radical, the other two substituents representing a hydrogen atom.

**4.** Process according to any one of claims 1 or 2, characterized in that a product of formula (II) is used at the start in which two of the three substituents $R_1$, $R_2$ or $R_3$ represent in position 9, 10 or 11 a chlorine atom, a methyl, ethyl, methoxy or ethoxy radical, the third substituent representing a hydrogen atom, or in that all three of $R_1$, $R_2$ and $R_3$ represent in these positions a chlorine atom or a methyl, ethyl, methoxy or ethoxy radical.

**5.** Process according to any one of claims 1 to 4, characterized in that a product of formula (II) is used at the start in which the hydrogen atom in position 3 and the hydrogen atom in position 16 are trans.

**6.** Process according to any one of claims 1 to 5, characterized in that a product of formula (II) is subjected to the action of an appropriate reducing agent in order to obtain a corresponding product of formula (I) in which

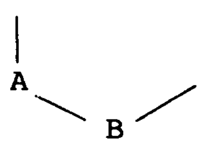

represents

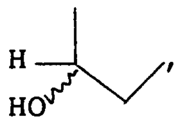

which if appropriate, is subjected to the action of an appropriate dehydration agent, in order to obtain a corresponding product of formula (I) in which

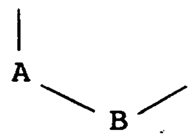

represents

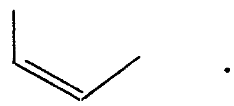

**7.** Process according to claim 1, characterized in that any one of the products whose names follow are prepared:
- [(+)(14alpha, 16alpha)] 14,15-dihydro 10-methoxy 20,21-dinoreburnamenin-14-ol,
- [(±)(14alpha, 16alpha)] 14,15-dihydro 11-methyl 20,21-dinoreburnamenin-14-ol,
- [(±)(16alpha)] 11-chloro 20,21-dinoreburnamenine,
- [(±)(16alpha)] 11-methoxy 20,21-dinoreburnamenine,
- [(±)(16alpha)] 11-methyl 20,21-dinoreburnamenine,

as well as their addition salts with mineral or organic acids.

**8.** Preparation process for pharmaceutical compositions, characterized in that at least one of the compounds of formula (I) as defined in claim 1 or at least one of their pharmaceutically acceptable salts is used as active ingredient in a form intended for this use.

**9.** Process according to claim 8, characterized in that the compound of formula (I) is chosen from the products whose names follow:
- [(+)(14alpha, 16alpha)] 14,15-dihydro 10-methoxy 20,21-dinoreburnamenin-14-ol,
- [(±)(14alpha, 16alpha)] 14,15-dihydro 11-methyl 20,21-dinoreburnamenin-14-ol,
- [(±)(16alpha)] 11-chloro 20,21-dinoreburnamenine,
- [(±)(16alpha)] 11-methoxy 20,21-dinoreburnamenine,
- [(±)(16alpha)] 11-methyl 20,21-dinoreburnamenine,

as well as their addition salts with mineral or organic acids.

**10.** The products of formula (II) as defined in claim 1 with the exception of the products of formula ($II_A$):

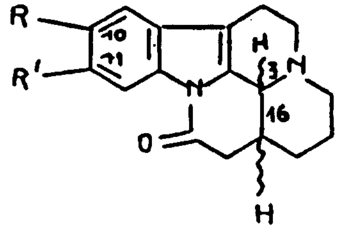

($II_A$)

for which R and R', identical or different, represent a hydroxy or methoxy radical.

**Patentansprüche**

**Patentansprüche für folgende Vertragsstaaten : AT, BE, CH, DE, FR, GB, IT, LI, LU, NL, SE**

1. Verbindungen der Formel (I):

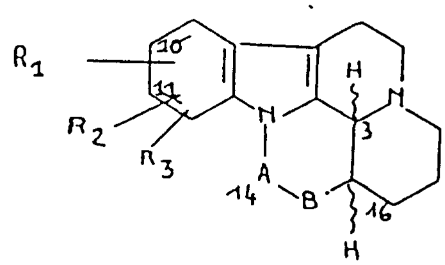

(I)

in der $R_1$, $R_2$ und $R_3$, die gleich oder verschieden sein können, ein Wasserstoffatom, ein Halogenatom, einen Alkyl- oder Alkoxyrest mit 1 bis 5 Kohlenstoffatomen, einen Hydroxy-, Trifluormethyl-, Nitro-, Amino-, Alkylamino-, Dialkylaminorest, in dem der Alkylrest 1 bis 5 Kohlenstoffatome einschließt, einen Acylaminorest, in dem der Acylrest ein Rest einer aliphatischen Säure mit bis zu 6 Kohlenstoffatomen ist, darstellen, wobei $R_1$, $R_2$ und $R_3$ nicht gleichzeitig ein Wasserstoffatom darstellen können, und in der die Gruppe:

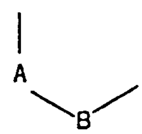

entweder für:

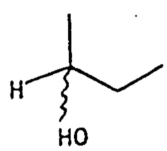

oder:

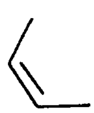

oder auch:

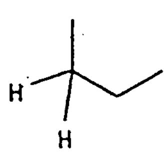

steht, wobei die Produkte der Formel (I) in allen möglichen racemischen oder optisch aktiven isomeren Formen vorliegen, sowie die Additionssalze der Produkte der Formel (I) mit Mineral- oder organischen Säuren.

**2.** Verbindungen der Formel (I), wie in Anspruch 1 definiert, dadurch gekennzeichnet, daß $R_1$, $R_2$ und $R_3$, die gleich oder verschieden sein können, ein Wasserstoffatom oder einen Methyl-, Ethyl-, Methoxy- oder Ethoxyrest, ein Chloratom, einen Hydroxy-, Trifluormethyl- oder Nitrorest darstellen, in allen möglichen isomeren Formen, racemisch oder optisch aktiv, sowie die Additionssalze mit Mineral- oder organischen Säuren.

**3.** Verbindungen der Formel (I), wie im Anspruch 1 oder 2 definiert, dadurch gekennzeichnet, daß einer der drei Substituenten $R_1$ oder $R_2$ oder $R_3$ in Stellung 10 oder 11 einen Methyl-, Ethyl-, Methoxy- oder Ethoxyrest, ein Chloratom, einen Hydroxy-, Trifluormethyl- oder Nitrorest darstellt, wobei die beiden anderen Substituenten ein Wasserstoffatom darstellen, in allen möglichen isomeren Formen, racemisch oder optisch aktiv, sowie die Additionssalze mit Mineral- oder organischen Säuren.

**4.** Verbindungen der Formel (I), wie in einem der Ansprüche 1 oder 2 definiert, dadurch gekennzeichnet, daß zwei der drei Substituenten $R_1$, $R_2$ oder $R_3$ in Stellung 9, 10 oder 11 ein Chloratom, einen Methyl-, Ethyl-, Methoxy- oder Ethoxyrest darstellen, wobei der dritte Substituent ein Wasserstoffatom darstellt, oder dadurch, daß $R_1$, $R_2$ und $R_3$ alle drei in diesen Stellungen ein Chloratom oder einen Methyl-, Ethyl-, Methoxy- oder Ethoxyrest darstellen, in allen möglichen isomeren Formen, racemisch oder optisch aktiv, sowie die Additionssalze mit Mineral- oder organischen Säuren.

**5.** Verbindungen der Formel (I), wie in einem der Ansprüche 1 bis 4 definiert, dadurch gekennzeichnet, daß das Wasserstoffatom in Stellung 3 und das Wasserstoffatom in Stellung 16 trans stehen, in allen möglichen racemischen oder optisch aktiven isomeren Formen, sowie die Additionssalze mit Mineral- oder organischen Säuren.

**6.** Verbindungen der Formel (I), wie in einem der Ansprüche 1 bis 5 definiert, dadurch gekennzeichnet, daß die Gruppe

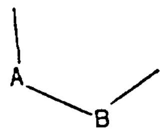

entweder

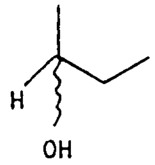

oder

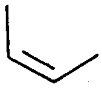

darstellt, in allen möglichen isomeren Formen, racemisch oder optisch aktiv, sowie die Additionssalze mit Mineral- oder organischen Säuren.

7. Verbindungen der Formel (I), deren Namen folgen:
[(+)(14alpha,16alpha)]-14,15-Dihydro-10-methoxy-20,21-dinoreburnamenin-14-ol,
[(±)(14alpha,16alpha)]-14,15-Dihydro-11-methyl-20,21-dinoreburnamenin-14-ol,
[(±)(16alpha)]-11-Chlor-20,21-dinoreburnamenin,
[(±)(16alpha)]-11-Methoxy-20,21-dinoreburnamenin,
[(±)(16alpha)]-11-Methyl-20,21-dinoreburnamenin,
sowie deren Additionssalze mit Mineral- oder organischen Säuren.

8. Verfahren zur Herstellung der Verbindungen der Formel (I), wie in Anspruch 1 definiert, dadurch gekennzeichnet, daß man eine Verbindung der Formel (II):

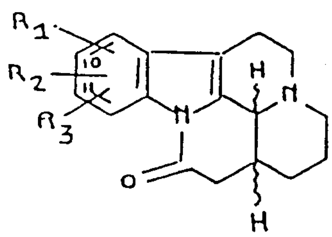

(II)

in der $R_1$, $R_2$ und $R_3$ die bereits angegebenen Bedeutungen aufweisen, reduziert, um eine Verbindung der Formel ($I_A$) zu erhalten, welche einem Produkt der Formel (I) entspricht, in der:

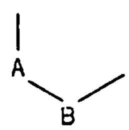

für

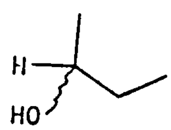

steht, wobei die Verbindung der Formel ($I_A$), falls gewünscht, dehydratisiert wird, um eine der Formel ($I_B$) entsprechende Verbindung zu erhalten, welche eine Verbindung der Formel (I) darstellt, in der

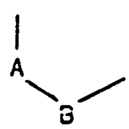

für

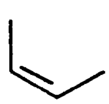

steht, wobei die Verbindung der Formel ($I_B$), falls gewünscht, zu einer der Formel ($I_C$) entsprechenden Verbindung reduziert wird, welche eine Verbindung der Formel (I) darstellt, in der:

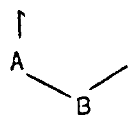

für

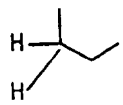

steht,
und, falls gewünscht, die erhaltenen Produkte der Formel (I) mit einer Mineral- oder organischen Säure behandelt, um daraus das Salz zu bilden.

**9.** Verbindungen, wie durch die Formel (I) des Anspruchs 1 definiert, wobei die Produkte der Formel (I) in allen möglichen isomeren Formen, racemisch oder optisch aktiv, vorliegen, sowie die pharmazeutisch annehmbaren Additionssalze mit Mineral- oder organischen Säuren, als Arzneimittel.

**10.** Verbindungen, wie in den Ansprüche 2 bis 6 definiert, in allen möglichen isomeren Formen, racemisch oder optisch aktiv, sowie die pharmazeutisch annehmbaren Additionssalze mit Mineral- oder organischen Säuren als Arzneimittel.

**11.** Verbindungen, wie in Anspruch 7 definiert, sowie deren pharmazeutisch annehmbare Additionssalze mit Mineral- oder organischen Säuren als Arzneimittel.

**12.** Pharmazeutische Zusammensetzungen, die als Wirkstoff mindestens ein wie in irgendeinem der Ansprüche 9 bis 11 definiertes Arzneimittel enthalten.

**13.** Produkte der Formel (II), wie in Anspruch 8 definiert, mit Ausnahme der Produkte der Formel ($II_A$):

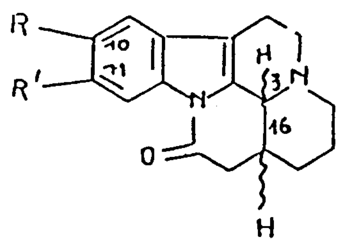

($II_A$)

für die R und R', die gleich oder verschieden sein können, einen Hydroxy- oder Methoxyrest darstellen.

**Patentansprüche für folgenden Vertragsstaat : ES**

1. Verfahren zur Herstellung von Verbindungen der Formel (I):

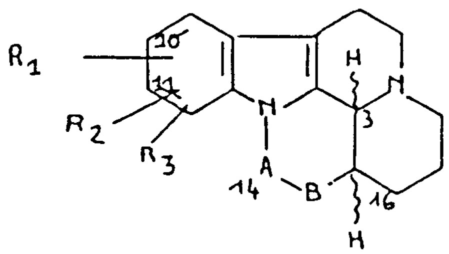

(I)

in der $R_1$, $R_2$ und $R_3$, die gleich oder verschieden sein können, ein Wasserstoffatom, ein Halogenatom, einen Alkyl- oder Alkoxyrest mit 1 bis 5 Kohlenstoffatomen, einen Hydroxy-, Trifluormethyl-, Nitro-, Amino-, Alkylamino-, Dialkylaminorest, in dem der Alkylrest 1 bis 5 Kohlenstoffatome einschließt, einen Acylaminorest, in dem der Acylrest ein Rest einer aliphatischen Säure mit bis zu 6 Kohlenstoffatomen ist, darstellen, wobei $R_1$, $R_2$ und $R_3$ nicht gleichzeitig ein Wasserstoffatom darstellen können, und in der die Gruppe:

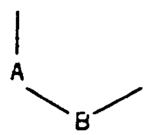

entweder für:

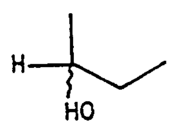

oder:

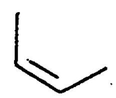

oder auch:

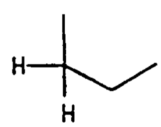

steht, wobei die Produkte der Formel (I) in allen möglichen racemischen oder optisch aktiven isomeren Formen vorliegen, sowie der Additionssalze der Produkte der Formel (I) mit Mineral- oder organischen Säuren,
dadurch gekennzeichnet, daß man eine Verbindung der Formel (II):

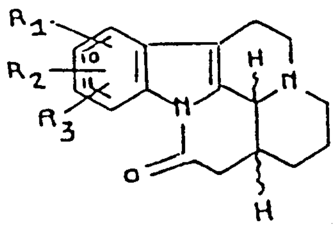

(II)

in der $R_1$, $R_2$ und $R_3$ die bereits angegebenen Bedeutungen aufweisen, reduziert, um eine Verbindung der Formel ($I_A$) zu erhalten, welche einem Produkt der Formel (I) entspricht, in der:

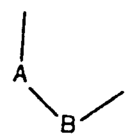

für

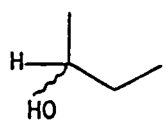

steht, wobei die Verbindung der Formel ($I_A$), falls gewünscht, dehydratisiert wird, um eine der Formel ($I_B$) entsprechende Verbindung zu erhalten, welche eine Verbindung der Formel (I) darstellt, in der

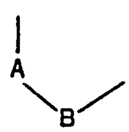

für

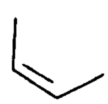

steht, wobei die Verbindung der Formel ($I_B$), falls gewünscht, zu einer der Formel ($I_C$) entsprechenden Verbindung reduziert wird, welche eine Verbindung der Formel (I) darstellt, in der:

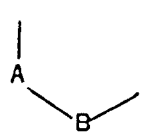

für

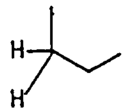

steht,
und, falls gewünscht, die erhaltenen Produkte der Formel (I) mit einer Mineral- oder organischen Säure behandelt, um daraus das Salz zu bilden.

2. Verfahren gemäß Anspruch 1, dadurch gekennzeichnet, daß man zu Beginn ein Produkt der Formel (II), in der $R_1$, $R_2$ und $R_3$, die gleich oder verschieden sein können, ein Wasserstoffatom oder einen Methyl-, Ethyl-, Methoxy- oder Ethoxyrest, ein Chloratom, einen Hydroxy-, Trifluormethyl- oder Nitrorest darstellen, verwendet.

3. Verfahren gemäß Anspruch 1 oder 2, dadurch gekennzeichnet, daß man zu Beginn ein Produkt der Formel (II), in der einer der drei Substituenten $R_1$ oder $R_2$ oder $R_3$ in Stellung 10 oder 11 einen Methyl-, Ethyl-, Methoxy- oder Ethoxyrest, ein Chloratom, einen Hydroxy-, Trifluormethyl- oder Nitrorest darstellt, wobei die beiden anderen Substituenten ein Wasserstoffatom darstellen, verwendet.

4. Verfahren gemäß irgendeinem der Ansprüche 1 oder 2, dadurch gekennzeichnet, daß man zu Beginn ein Produkt der Formel (II), in der zwei der drei Substituenten $R_1$, $R_2$ oder $R_3$ in Stellung 9, 10 oder 11 ein Chloratom, einen Methyl-, Ethyl-, Methoxy- oder Ethoxyrest darstellen, wobei der dritte Substituent ein Wasserstoffatom darstellt, oder dadurch, daß $R_1$, $R_2$ und $R_3$ alle drei in diesen Stellungen ein Chloratom oder einen Methyl-, Ethyl-, Methoxy- oder Ethoxyrest darstellen, verwendet.

5. Verfahren gemäß irgendeinem der Ansprüche 1 bis 4, dadurch gekennzeichnet, daß man zu Beginn ein Produkt der Formel (II), in der das Wasserstoffatom in Stellung 3 und das Wasserstoffatom in Stellung 16 trans stehen, verwendet.

6. Verfahren nach irgendeinem der Ansprüche 1 bis 5, dadurch gekennzeichnet, daß man ein Produkt der Formel (II) der Einwirkung eines geeigneten Reduktionsmittels unterzieht, um ein Produkt zu erhalten, welches der Formel (I) entspricht, in der

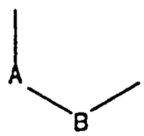

für

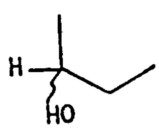

steht,
welches man gegebenenfalls der Einwirkung eines geeigneten Dehydratisierungsmittels unterwirft, um ein Produkt zu erhalten, welches der Formel (I) entspricht, in der

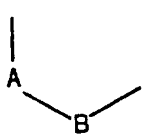

für

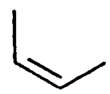

steht.

**7.** Verfahren gemäß Anspruch 1, dadurch gekennzeichnet, daß man irgendeines der Produkte, deren Namen folgen:
[(+)(14alpha,16alpha)]-14,15-Dihydro-10-methoxy-20,21-dinoreburnamenin-14-ol,
[(±)(14alpha,16alpha)]-14,15-Dihydro-11-methyl-20,21-dinoreburnamenin-14-ol,
[(±)(16alpha)]-11-Chlor-20,21-dinoreburnamenin,
[(±)(16alpha)]-11-Methoxy-20,21-dinoreburnamenin,
[(±)(16alpha)]-11-Methyl-20,21-dinoreburnamenin,
sowie deren Additionssalze mit Mineral- oder organischen Säuren herstellt.

**Patentansprüche für folgenden Vertragsstaat : GR**

**1.** Verfahren zur Herstellung von Verbindungen der Formel (I):

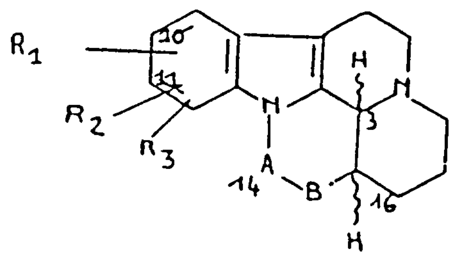

(I)

in der $R_1$, $R_2$ und $R_3$, die gleich oder verschieden sein können, ein Wasserstoffatom, ein Halogenatom, einen Alkyl- oder Alkoxyrest mit 1 bis 5 Kohlenstoffatomen, einen Hydroxy-, Trifluormethyl-, Nitro-, Amino-, Alkylamino-, Dialkylaminorest, in dem der Alkylrest 1 bis 5 Kohlenstoffatome einschließt, einen Acylaminorest, in dem der Acylrest ein Rest einer aliphatischen Säure mit bis zu 6 Kohlenstoffatomen ist, darstellen, wobei $R_1$, $R_2$ und $R_3$ nicht gleichzeitig ein Wasserstoffatom darstellen können, und in der die Gruppe:

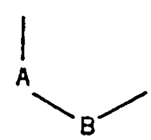

entweder für:

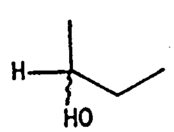

oder:

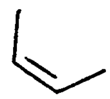

oder auch:

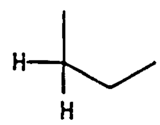

steht, wobei die Produkte der Formel (I) in allen möglichen racemischen oder optisch aktiven isomeren Formen vorliegen, sowie der Additionssalze der Produkte der Formel (I) mit Mineral- oder organischen Säuren,
dadurch gekennzeichnet, daß man eine Verbindung der Formel (II):

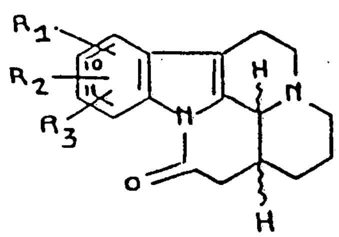

(II)

in der $R_1$, $R_2$ und $R_3$ die bereits angegebenen Bedeutungen aufweisen, reduziert, um eine Verbindung der Formel ($I_A$) zu erhalten, welche einem Produkt der Formel (I) entspricht, in der:

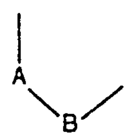

für

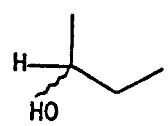

steht, wobei die Verbindung der Formel ($I_A$), falls gewünscht, dehydratisiert wird, um eine der Formel ($I_B$) entsprechende Verbindung zu erhalten, welche eine Verbindung der Formel (I) darstellt, in der

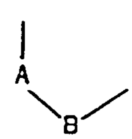

für

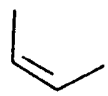

steht, wobei die Verbindung der Formel ($I_B$), falls gewünscht, zu einer der Formel ($I_C$) entsprechenden Verbindung reduziert wird, welche eine Verbindung der Formel (I) darstellt, in der:

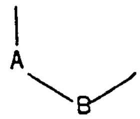

für

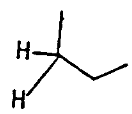

steht,
und, falls gewünscht, die erhaltenen Produkte der Formel (I) mit einer Mineral- oder organischen Säure behandelt, um daraus das Salz zu bilden.

**2.** Verfahren gemäß Anspruch 1, dadurch gekennzeichnet, daß man zu Beginn ein Produkt der Formel (II), in der $R_1$, $R_2$ und $R_3$, die gleich oder verschieden sein können, ein Wasserstoffatom oder einen Methyl-, Ethyl-, Methoxy- oder Ethoxyrest, ein Chloratom, einen Hydroxy-, Trifluormethyl- oder Nitrorest darstellen, verwendet.

**3.** Verfahren gemäß Anspruch 1 oder 2, dadurch gekennzeichnet, daß man zu Beginn ein Produkt der Formel (II), in der einer der drei Substituenten $R_1$ oder $R_2$ oder $R_3$ in Stellung 10 oder 11 einen Methyl-, Ethyl-, Methoxy- oder Ethoxyrest, ein Chloratom, einen Hydroxy-, Trifluormethyl- oder Nitrorest darstellt, wobei die beiden anderen Substituenten ein Wasserstoffatom darstellen, verwendet.

**4.** Verfahren gemäß irgendeinem der Ansprüche 1 oder 2, dadurch gekennzeichnet, daß man zu Beginn ein Produkt der Formel (II), in der zwei der drei Substituenten $R_1$, $R_2$ oder $R_3$ in Stellung 9, 10 oder 11 ein Chloratom, einen Methyl-, Ethyl-, Methoxy- oder Ethoxyrest darstellen, wobei der dritte Substituent ein Wasserstoffatom darstellt, oder dadurch, daß $R_1$, $R_2$ und $R_3$ alle drei in diesen Stellungen ein Chloratom oder einen Methyl-, Ethyl-, Methoxy- oder Ethoxyrest darstellen, verwendet.

**5.** Verfahren gemäß irgendeinem der Ansprüche 1 bis 4, dadurch gekennzeichnet, daß man zu Beginn ein Produkt der Formel (II), in der das Wasserstoffatom in Stellung 3 und das Wasserstoffatom in Stellung 16 trans stehen, verwendet.

**6.** Verfahren nach irgendeinem der Ansprüche 1 bis 5, dadurch gekennzeichnet, daß man ein Produkt der Formel (II) der Einwirkung eines geeigneten Reduktionsmittels unterzieht, um ein Produkt zu erhalten, welches der Formel (I) entspricht, in der

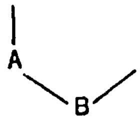

für

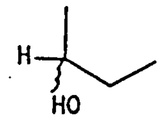

steht,
welches man gegebenenfalls der Einwirkung eines geeigneten Dehydratisierungsmittels unterwirft, um ein Produkt zu erhalten, welches der Formel (I) entspricht, in der

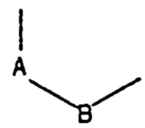

für

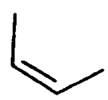

steht.

7. Verfahren gemäß Anspruch 1, dadurch gekennzeichnet, daß man irgendeines der Produkte, deren Namen folgen:
[(+)(14alpha,16alpha)]-14,15-Dihydro-10-methoxy-20,21-dinoreburnamenin-14-ol,
[(±)(14alpha,16alpha)]-14,15-Dihydro-11-methyl-20,21-dinoreburnamenin-14-ol,
[(±)(16alpha)]-11-Chlor-20,21-dinoreburnamenin,
[(±)(16alpha)]-11-Methoxy-20,21-dinoreburnamenin,
[(±)(16alpha)]-11-Methyl-20,21-dinoreburnamenin,
sowie deren Additionssalze mit Mineral- oder organischen Säuren herstellt.

8. Verfahren zur Herstellung von pharmazeutischen Zusammensetzungen, dadurch gekennzeichnet, daß man als Wirkstoff mindestens eine der wie in Anspruch 1 definierten Verbindungen der Formel (I) oder mindestens eines der pharmazeutisch annehmbarer Salze derselben in eine für diese Verwendung bestimmte Form bringt.

9. Verfahren nach Anspruch 8, dadurch gekennzeichnet, daß die Verbindung der Formel (I) ausgewählt ist aus den Produkten, deren Namen folgen:
[(+)(14alpha,16alpha)]-14,15-Dihydro-10-methoxy-20,21-dinoreburnamenin-14-ol,
[(±)(14alpha,16alpha)]-14,15-Dihydro-11-methyl-20,21-dinoreburnamenin-14-ol,
[(±)(16alpha)]-11-Chlor-20,21-dinoreburnamenin,
[(±)(16alpha)]-11-Methoxy-20,21-dinoreburnamenin,
[(±)(16alpha)]-11-Methyl-20,21-dinoreburnamenin,
sowie deren Additionssalzen mit Mineral- oder organischen Säuren.

**10.** Produkte der Formel (II), wie in Anspruch 1 definiert, mit Ausnahme der Produkte der Formel ($II_A$):

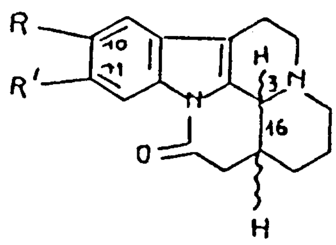

($II_A$)

für die R und R', die gleich oder verschieden sein können, einen Hydroxy- oder Methoxyrest darstellen.